# EUROPEAN PATENT SPECIFICATION

(11) **EP 2 032 128 B1**
(45) Date of publication and mention of the grant of the patent: **15.08.2012**
(21) Application number: 07726074.3
(22) Date of filing: 19.06.2007
(51) Int. Cl.: A61K 31/135, A61K 31/381, A61P 29/00

(54) **USE OF SUBSTITUTED 2-AMINOTETRALINES FOR THE MANUFACTURE OF A MEDICAMENT FOR THE PREVENTION, ALLEVIATION AND/OR TREATMENT OF VARIOUS TYPES OF PAIN**
VERWENDUNG VON SUBSTITUIERTEN 2-AMINOTETRALINEN ZUR HERSTELLUNG EINES MEDIKAMENTS ZUR PRÄVENTION, LINDERUNG UND/ODER BEHANDLUNG VON VERSCHIEDENEN SCHMERZARTEN
UTILISATION DE 2-AMINOTÉTRALINES SUBSTITUÉES POUR LA FABRICATION D'UN MÉDICAMENT DESTINÉ À PRÉVENIR, SOULAGER ET/OU TRAITER DIVERS TYPES DE DOULEUR

(30) Priority: 22.06.2006 EP 06012815
(43) Date of publication of application: 11.03.2009
(73) Proprietor: UCB Pharma GmbH, 40789 Monheim (DE)
(72) Inventor: BEYREUTHER, Bettina, 40219 Düsseldorf (DE); SCHELLER, Dieter, 41470 Neuss (DE); FREITAG, Joachim, 72622 Nürtingen (DE); BIANCHINE, Joseph, Potomac, Maryland 20854 (US)
(74) Representative: HOFFMANN EITLE
(86) International application number: PCT/EP2007/005381
(87) International publication number: WO 2007/147556

(56) References cited:
- WO-A-03/092677
- WO-A-2005/009425
- WO-A-2006/032202
- US-A- 5 153 225

## Description

### Field of the Invention

The present invention relates to the use of compounds of the formula (I) 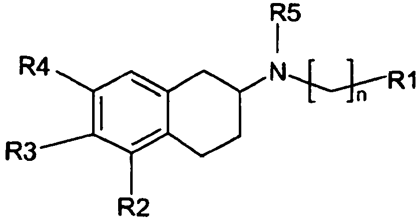 in which R1, R2, R3, R4, R5 and n have the meanings indicated below, for the preparation of a pharmaceutical composition for the prevention, alleviation and/or treatment of various types of pain.

### Background of the Invention

Pain is a complex physiological process that involves a number of sensory and neural mechanisms. Acute pain is typically a physiological signal indicating a potential or actual injury. Chronic pain can be somatogenetic (organic) or psychogenic. Chronic pain is frequently accompanied or followed by vegetative signs such as for example lassitude or sleep disturbance.

Somatogenetic pain may be of nociceptive, inflammatory or neuropathic origin. Nociceptive pain is related to activation of somatic or visceral pain-sensitive nerve fibers, typically by physical or chemical injury of tissues. Inflammatory pain results from inflammation, for example an inflammatory response of living tissues to any stimulus including injury, infection or irritation. Neuropathic pain results from dysfunction in the nervous system. Neuropathic pain is believed to be sustained by aberrant somatosensory mechanisms in the peripheral nervous system, the central nervous system (CNS), or both.

Non-inflammatory musculoskeletal pain is a particular form of chronic pain that is generally not traced to a specific structural or inflammatory cause and that generally does not appear to be induced by tissue damage and macrophage infiltration (resulting in edema) as occurs in a classical immune system response.

Although non-inflammatory musculoskeletal pain is believed to result from peripheral and/or central sensitization, the cause is not presently fully understood. It is often associated with physical or mental stress, lack of adequate or restful sleep, or exposure to cold or damp. Non-inflammatory musculoskeletal pain is also believed to be associated with or precipitated by systemic disorders such as viral or other infections. Examples of non-inflammatory musculoskeletal pain include neck and shoulder pain and spasms, low back pain, and achy chest or thigh muscles. Non-inflammatory musculoskeletal pain may be generalized or localized. Understanding of the basic causes and mechanisms, the animal and other models for studying non-inflammatory musculoskeletal pain, and treatment regimens are all areas where a need for improvement exists.

Fibromyalgia syndrome (FMS) and myofascial pain syndrome (MPS) are medical conditions characterized by fibromyalgia and myofascial pain respectively, which are two types of non-inflammatory musculoskeletal pain.

FMS is a complex syndrome associated with significant impairment of quality of life and can result in substantial financial costs. Fibromyalgia is a systemic process that typically causes tender points (local tender areas in normal-appearing tissues) in particular areas of the body and is frequently associated with a poor sleep pattern and/or stressful environment. The diagnosis of fibromyalgia is typically based on a history of widespread pain (*e.g.* bilateral, upper and lower body, and/or the spine), and the presence of excessive tenderness on applying pressure to a number of (sometimes more precisely defined as at least 11 out of 18) specific muscle-tender sites. FMS is typically a chronic syndrome that causes pain and stiffness throughout the tissues that support and move the bones and joints.

Treatment of fibromyalgia is conventionally based on pain-relievers, non-steroidal anti-inflammatory drugs (NSAIDs), muscle relaxants, tranquilizers and anti-depressant drugs, none of which are universally effective. Fibromyalgia patients often sleep poorly and may experience some relief by taking the antidepressant such as amitriptyline at bedtime. See Goldenberg DL, Burckhardt C, Crofford L., et al.," JAMA 292(19):2388-95 (2004). A goal in treating fibromyalgia is to decrease pain and to increase function. Fibromyalgia has been reviewed, for example by Nampiaparampil & Shmerling, Am. J. Manag. Care, 10(11 Pt 1):794-800 (2004).

Myofascial pain syndrome (MPS) is a chronic non-degenerative, non-inflammatory musculoskeletal pain condition often associated with spasm or pain in the masticatory muscles. Distinct areas within muscles or their delicate connective tissue coverings (fascia) become abnormally thickened or tight. When the myofascial tissues tighten and lose their elasticity, the ability of neurotransmitters to send and receive messages between the brain and body is disrupted. Specific discrete areas of muscle may be tender when firm fingertip pressure is applied; these areas are called either tender or trigger points. (Both points are tender, but "trigger" points additionally radiate the pain to a distant site.) Symptoms of MPS include muscle stiffness and aching and sharp shooting pains or tingling and numbness in areas distant from the trigger point. The discomfort may cause sleep disturbance, fatigue, and depression. Most commonly trigger points are in the jaw (temporomandibular region), neck, back, or buttocks.

Myofascial pain differs from fibromyalgia: MPS and FMS are two separate entities, each having its own pathology, but sharing the muscle as a common pathway of pain. Myofascial pain is typically a more localized or regional (along the muscle and surrounding fascia tissues) pain process that is often associated with trigger point tenderness. Myofascial pain can be treated with a variety of methods (sometimes in combination) including stretching, ultrasound, ice sprays with stretching, exercises, and injections of anesthetic.

A further non-inflammatory musculoskeletal pain condition is back pain, notably low back pain. Back pain is a common musculoskeletal symptom that may be either acute or chronic. It may be caused by a variety of diseases and disorders that affect the lumbar spine. Low back pain is often accompanied by sciatica, which is pain that involves the sciatic nerve and is felt in the lower back, the buttocks, and the backs of the thighs.

Non-inflammatory musculoskeletal pain such as fibromyalgia, myofascial pain, and back pain involves increased muscle sensitivity as an important manifestation. Increased muscle sensitivity is characterized by pain evoked by a normally non-nociceptive stimulus (allodynia) or increased pain intensity evoked by nociceptive stimuli (hyperalgesia).The term "allodynia" refers to a normally innocuous somatosensory stimulation that evokes abnormal intense pain sensation with an explosive, radiating character often outlasting stimulus or trigger duration (i.e. pain due to a stimulus that does not normally provoke pain). The term "hyperalgesia" refers to an noxious stimulation that evokes more intense and prolonged pain sensations (*i.e.* an increased response to a stimulus that is normally painful).

Two classes of drugs are generally employed for the treatment of various types of pain: non-opioid analgesics, including acetaminophen and NSAIDs, and opioid (narcotic) analgesics. Both opioids and non-opioids have several unwanted side effects. The most serious effects of opioids are the possibility of inhibition of the respiratory system and, after long-term treatment, the possibility of addiction. NSAIDs, on the other hand, can induce a variety of gastrointestinal complications such as ulcers and bleeding, but also kidney damage.

In part because of such side effects, alternative drug therapies have been proposed for treatment of pain. Such drugs include anticonvulsants, antidepressants, serotonin modulators, norepinephrine re-uptake inhibitors, dopamine agonists and combinations thereof.

For example, U.S. Patent No. 5,658,955 proposes use of a combination of a serotonin agonist and a dopamine agonist to treat fibromyalgia, among other conditions. Phentermine is described therein as a preferred dopamine agonist.

U.S. Patent No. 5,872,127 proposes treatment of a variety of diseases, including fibromyalgia, through management of prolactin levels using a serotonin agonist and a dopamine agonist.

U.S. Patent No. 6,448,258 to McCall et al. proposes treatment of fibromyalgia syndrome or chronic fatigue syndrome with compounds said to have dopamine receptor activity, including cabergoline.

The publications individually cited below each propose a method for the treatment of human patients afflicted with fibromyalgia, using non-ergot dopamine receptor agonists which are tetrahydrobenzothiazole and 3(H)-indolone compounds, illustratively pramipexole and ropinirole respectively.
- International Patent Publication No. WO02/05797
- U.S. Patent No. 6,277,875.
- U.S. Patent No. 6,300,365.
- Holman, J. Muscoloskeletal Pain, 12(1):69-74 (2004).

Pramipexole and ropinirole are non-ergolinic agonists of the D2 subfamily of dopamine recptors (D2, D3, and D4), having strongest affinity for D3. They show only weak or no affinity for D1, for 5-hydroxytryptamine (5-HT) receptors such as 5-HT_{1A} and 5-HT₇, or for alpha-adrenergic receptors suchs as α2B or α2C. Pramipexole and ropinirole have been shown to reduce pain in preliminary clinical studies with fibromyalgia patients (Holman, Arthritis & Rheumatism, 52(8):2495-2505 (2005); Holman, Arthritis & Rheumatism, 50 (2004) Suppl. 9:S698).

These dopamine agonists are known to commonly lead, usually in the beginning of therapy and as a function of the dosage administered, to various side effects, including, for example, psychiatric, neurological, vascular and gastrointestinal effects. Psychiatric effects reported for pramipexole or ropinirole have included insomnia, hallucinations and confusion. Neurological effects have included syncope of fainting, somnolence, dizziness or vertigo, and dyskinesia. Gastrointestinal effects have included vomiting, nausea, abdominal pain, constipation and heartbum.

Attacks of drowsiness have been described as a serious side effect of pramipexole. For side effects of pramipexole, see, for example, a Scientific Discussion posted by the European Medicines Agency at http://www.emea.eu.int/humandocs/PDFs/EPAR/Sifrol/059197EN6.pdf.

International patent application WO 2006/032202 discloses the use of dopamine receptor agonists and in particular 2-aminotretralins including rotigotine in the treatment of Parkinson's disease thereby alleviating the suffering of Parkinson's disease patients and improving their quality of life.

European patent application EP 1 797 871 proposes treatment of Parkinson's disease with dopamine receptor agonists and in particular 2-aminotretralins including rotigotine thereby alleviating the pain of patients with Parkinsons disease and improving their quality of life.

International patent application WO 2005/009425 discloses the use of 2-aminotetralins including rotigotine in the treatment of depression, which may *inter alia* be classified as organic depression caused by an underlying brain disease or injury such as migraine.

International patent application WO 03/092677 proposes treatment of Restless Leg Syndrome (RLS) with rotigotine. Among the symptoms of RLS pain is disclosed in WO 03/092677.

There is a continuing need to provide alternative treatments having therapeutic efficacy in the treatment of chronic and/or acute pain, in particular systemic treatment of non-inflammatory musculoskeletal pain, including fibromyalgia, myofascial pain or back pain and associated muscular hyperalgesia and allodynia.

Therefore, it is an objective of the present invention to provide alternative medicines for the treatment of chronic and/or acute pain and especially of non-inflammatory musculoskeletal pain, in particular fibromyalgia, myofascial pain or back pain. Specifically, it is an objective of the invention to provide a treatment, preferably a systemic treatment, of non-inflammatory musculoskeletal pain including fibromyalgia, myofascial pain or back pain which are characterized by increased pain intensity evoked by nociceptive stimuli (hyperalgesia) and/or by increased pain intensity evoked by normally non-nociceptive stimuli (allodynia) in the absence of a physiological cause such as inflammatory edema.

It has now been surprisingly found that rotigotine, representative of compounds of Formula (I) below, has analgesic potency and can be used to treat (including to prevent and/or alleviate) various types of pain. The compounds of the present invention are in particular able to reduce antinociceptive behavior in patients suffering from, or in anticipation of, non-inflammatory musculoskeletal pain such as back pain, fibromyalgia, myofascial pain and in particular in reducing the associated muscular hyperalgesia or muscular allodynia.

### Summary of the Invention

In one aspect, the present invention involves substituted 2-aminotetralines according to Formula (I) 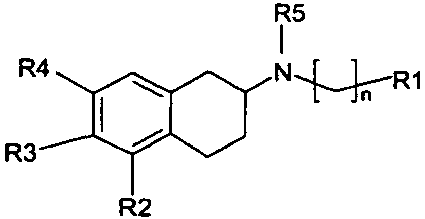 wherein
n is a number from 1 to 5;
R1 is selected from the group consisting of hydrogen, 3-pyridyl, 4-pyridyl, optionally substituted phenyl, 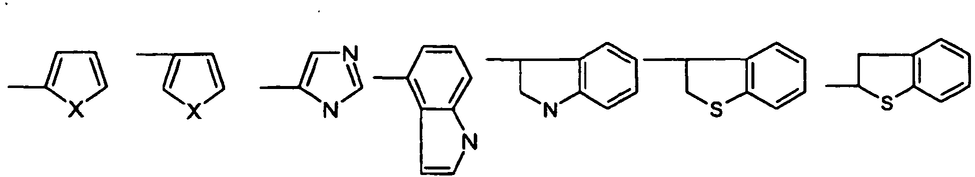 wherein X is S, O or NH;
R2 is a group -OA; and R3 and R4 are each independently hydrogen or a group -OA; wherein A is hydrogen, alkyl, in particular C₁₋₆ alkyl, cycloalkyl, in particular C₃₋₁₀ cycloalkyl, aryl, in particular optionally substituted phenyl, alkoxyalkyl, in particular alkoxy-C₁₋₆ alkyl, more particularly alkoxy-C₁₋₃ alkyl, for example alkoxymethyl, -C(=S)R6, -C(=S)OR6, -C(=S)NR6R7, for example -C(=S)NHR6 or -C(=S)NH₂, -S(O)₂R6, -S(O)₂OR6, -P(O₂H)R6, -P(O₂H)OR6, -CHR6OC(O)R7, -C₁₋₃ alkyl-OC(O)R6, in particular -CH₂-OC(O)R6, -C(OR6)R7R8, -CH(OR6)R7, - C(O)R6, -C(O)NR6R7, for example -C(O)NHR6 or C(O)NH₂, or -C(O)OR6, wherein R6, R7 and R8 are each independently hydrogen, alkyl, in particular C₁₋₂₀ alkyl and more particularly C₁₋₁₂ alkyl and for example C₁₋₆ alkyl, cycloalkyl, in particular C₃₋₁₀ cycloalkyl and more particularly C₄₋₈ cycloalkyl and for example C₄₋₆ cycloalkyl, or aryl, in particular optionally substituted phenyl, and wherein alkyl substituents are optionally substituted with one or more halogen atoms; R5 is a C₁₋₃ alkyl;
wherein the compounds of Formula (I) can be pure enantiomers (R or S) or any mixture thereof, including racemates, or pharmaceutically acceptable salts, or metabolite thereof. The compounds are used for the preparation of a pharmaceutical composition for the prevention, alleviation and/or treatment of chronic and/or acute pain, preferably musculoskeletal pain, more preferably non-inflammatory musculoskeletal pain such as back pain, fibromyalgia, and myofascial pain, and in particular in reducing the associated muscular hyperalgesia or muscular allodynia. Most preferably the compounds of Formula (I) are used to prepare a medicament for the treatment of fibromyalgia.

Compounds that are particularly suitable include substituted 2-aminotetralines according to Formula (I), wherein R2 is an -OA group and R3 and R4 are independently hydrogen or an -OA group, it being particularly preferred for A to be a hydrogen or a group 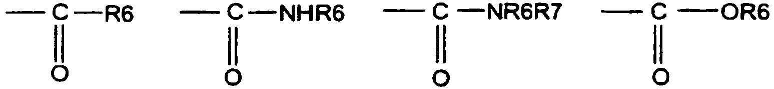 in which R6 and R7 are each independently C₁₋₂₀ alkyl, in particular C₁₋₁₂ alkyl more particularly C₁₋₆ alkyl, phenyl or methoxyphenyl. For example, R2 can be - OH or -OC(O)CH₃.

In another preferred embodiment of the present invention R3 is hydrogen.

In another preferred embodiment of the present invention R4 is hydrogen.

In another preferred embodiment of the present invention R3 and R4 are each hydrogen

In another preferred embodiment of the present invention n is 1, 2 or 3, preferably n = 2 or 3, for example 2.

In another preferred embodiment of the present invention R3 and R4 are hydrogen; R2 is -OH or -OC(O)CH₃ and n is 2.

R1 is preferably selected from the group 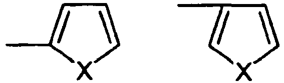 wherein X is selected from S, O and NH and wherein X is especially preferably a sulfur atom.

In a preferred embodiment R1 is 2-thienyl.

In a further preferred embodiment of the invention R5 is C₁₋₃ alkyl, for example a C₃ alkyl, in particular n-propyl.

In a further preferred embodiment of the invention, R1 is a 2-thienyl, R3 and R4 are both hydrogen, R5 is a C₃ alkyl and n = 2.

In one embodiment, a use of the invention comprises administering a compound of Formula (I) 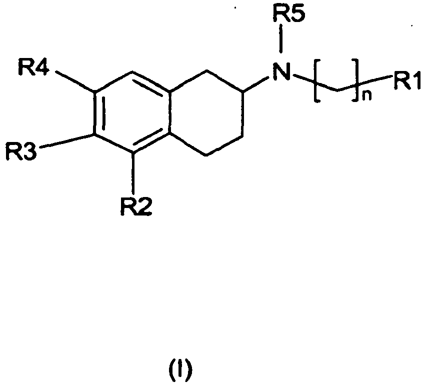 or an enantiomer, mixture of enantiomers, pharmaceutically acceptable salt, or metabolite thereof, wherein:
n is a number from 1 to 3;
R1 is 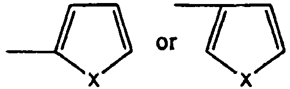 wherein X is O, S or NH;
R2 is a group -OA, and R3 and R4 are each independently hydrogen or a group -OA, wherein A is hydrogen or a group 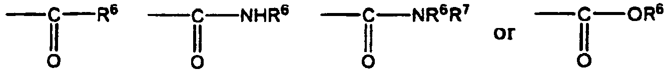
wherein R6 and R7 are each independently a C₁₋₂₀ alkyl (in particular C₁₋₁₂ alkyl, more particularly C₁₋₆ alkyl), phenyl or methoxyphenyl group; and R5 is C₁₋₃ alkyl.

In a particular embodiment, n is 2; R1 is 2-thienyl; R2 is hydroxyl, R3 and R4 are each hydrogen, and R5 is n-propyl. The compound of Formula (I) in this case is 5,6,7,8-Tetrahydro-6-[propyl[2-(2-thienyl)ethyl]amino]-1-naphthol or an enantiomer, mixture of enantiomers, pharmaceutically acceptable salt, or metabolite thereof.

Compounds of Formula (I), where optically active as in the case of 5,6,7,8-tetrahydro-6-[propyl[2-(2-thienyl)ethyl]amino]-1-naphthol, can be present as mixtures of enantiomers, for example racemates, or as pure (R)- or (S)-enantiomers. The term "pure enantiomer" herein means that at least about 90 mol % of the compound in question is present in the form of one enantiomer, *e.g.* in the (S) form, while the proportion of the respective other enantiomer, *e.g.* the (R) form, is correspondingly low.

Rotigotine (SPM962) is the (S)-(-)-enantiomer of 5,6,7,8-tetrahydro-6-[propyl[2-(2-thienyl)ethyl]amino]-1-naphthol. Rotigotine used according to the present invention typically is the pure (S)-(-)-enantiomer, the corresponding (R)-(+)-enantiomer is typically present in a proportion of less than about 10 mol %, more particularly less than about 2 mol %, for example less than about 1 mol %, of the total amount of 5,6,7,8-tetrahydro-6-[propyl[2-(2-thienyl)ethyl]amino]-1-naphthol in the pharmaceutical composition.

Compounds of Formula (I) can be present as free bases and/or in the form of pharmaceutically acceptable salts, *e.g.*, rotigotine in the form of rotigotine hydrochloride. Pharmaceutically acceptable salts include non-toxic addition salts of a compound of Formula (I) with organic or inorganic acids. Examples of inorganic acids include HCl.

The terms "C₁₋₂₀ alkyl", "C₁₋₁₂ alkyl", "C₁₋₆ alkyl" and "C₁₋₃ alkyl" as used herein mean, independently from each other, branched or unbranched alkyl groups with a total number of carbon atoms in the corresponding range. A "C₁₋₂₀ alkyl" group has, for example, 1 to 20 carbon atoms (whenever it appears herein, a numerical range such as "1 to 20" refers to each integer in the given range; *e.g.* "1 to 20 carbon atoms" means that the alkyl group may consist of 1 carbon atom, 2 carbon atoms, 3 carbon atoms, etc., up to and including 20 carbons.) The alkyl groups can optionally be substituted *e.g.* with halogen. In a particular preferred embodiment the alkyl groups are unsubstituted.

The term "cycloalkyl" when used alone or in combination is a cycloalkyl group containing from 3 to 18 ring carbon atoms and up to a total of 25 carbon atoms. Cycloalkyl groups may be monocyclic, bicyclic, tricyclic, or polycyclic and the rings can be fused. The cycloalkyl may be completely or partially saturated. Examples include cyclopropyl, cyclobutyl, cyclopentyl, cyclohexyl, cycloheptyl, cyclooctyl, cyclodecyl, cyclohexenyl, cyclopentenyl, cyclooctenyl, cycloheptenyl, decalinyl, hydroindanyl, indanyl, fenchyl, pinenyl, adamantyl, and the like. Cycloalkyl includes the cis or trans forms. Cycloalkyl groups may be unsubstituted or mono or polysubstituted with electron withdrawing and/or electron donating groups as described below. Furthermore, such substituents if present may be in endo- or exo- positions in bridged bicyclic systems. Illustrative cycloalkyls according to this inventions include those with 3 to 10 ring carbon atoms, particularly preferred are rings with 4 to 8 ring carbon atoms, even more particularly preferred are rings with 4 to 6 ring carbon atoms.

The term "alkoxy" herein means lower alkoxy containing from 1 to 6 carbon, especially 1 to 3 carbon atoms, and may be straight chain or branched. These groups include methoxy, ethoxy, propoxy, butoxy, isobutoxy, tert-butoxy, pentoxy, hexoxy and the like.

The term "aryl", when used alone or in combination, refers to an aromatic group which contains from 6 up to 18 ring carbon atoms and up to a total of 25 carbon atoms, and includes the polynuclear aromatics. Aryl groups may be monocyclic, bicyclic, tricyclic or polycyclic and can comprise fused rings. Polynuclear aromatic groups herein encompass bicyclic and tricyclic fused aromatic ring systems containing from 10 to 18 ring carbon atoms and up to a total of 25 carbon atoms. Aryl groups include phenyl, and polynuclear aromatic groups such as naphthyl, anthracenyl, phenanthrenyl, azulenyl and the like, and also include groups such as ferrocenyl. Aryl groups may be unsubstituted or monoor polysubstituted with electron-withdrawing and/or electron-donating groups as described below. In one embodiment an aryl group is a unsubstituted or monoor polysubstituted phenyl group. In a preferred embodiment the aryl group is a phenyl group.

The term "electron-withdrawing and "electron-donating" refer to the ability of a substituent to withdraw or donate electrons, respectively, relative to that of hydrogen if a hydrogen atom occupied the same position in the molecule. These terms are well understood by one skilled in the art and are discussed in Advanced Organic Chemistry, New York John Wiley and Sons, (1985), at pp.16-18, the disclosure of which is incorporated herein by reference. Electron-withdrawing groups include halo (including bromo, fluoro, chloro, iodo), nitro, carboxy, lower alkenyl, lower alkynyl, formyl, carboxyamido, aryl, quaternary ammonium, haloalkyl (such as trifluoromethyl), aryl lower alkanoyl, carbalkoxy and the like. Electron-donating groups include hydroxy, lower alkoxy (including methoxy, ethoxy, and the like), lower alkyl (including methyl, ethyl, and the like), amino, lower alkylamino, di(lower alkyl) amino, aryloxy (such as phenoxy), mercapto, lower alkylthio, lower alkylmercapto, disulfide (lower alkyldithio), and the like. One of ordinary skill in the art will appreciate that some of the aforesaid substituents may be considered to be electron-donating or electron-withdrawing under different chemical conditions. Moreover, the present invention contemplates any combination of substituents selected from the above-identified groups.

Illustrative electron-donating substituents and/or electron-withdrawing substituents are halo, nitro, alkanoyl, formyl, arylalkanoyl, aryloyl, carboxyl, carbalkoxy, carboxamido, cyano, sulfonyl, sulfoxide, heterocyclic, guanidine, quaternary ammonium, lower alkenyl, lower alkynyl, sulfonium salts, hydroxy, lower alkoxy, lower alkyl, amino, lower alkylamino, di(lower alkyl) amino, amino lower alkyl, mercapto, mercaptoalkyl, alkylthio, and alkyldithio. The term "sulfide" encompasses mercapto, mercapto alkyl and alkylthio, while the term disulfide encompasses alkyldithio. Particular examples of electron-donating and/or electron-withdrawing groups are halo and lower alkoxy, such as fluoro or methoxy.

In a further embodiment, the compound administered is a metabolite of a compound of Formula (I), for example such a compound wherein R1 is 2-thienyl, R3 and R4 are each hydrogen, R5 is C₃ alkyl, n is 2, and R2 is -OA, wherein A is a chemical moiety as defined above, more particularly wherein the active compound is rotigotine. An example of such a metabolite of rotigotine is (S)-2-N-propylamino-5-hydroxytetralin, as disclosed for example in International Patent Publication No. WO 2005/058296, incorporated herein by reference.

Substituted 2-aminotetralin compounds useful herein, such as rotigotine, can be prepared in a conventional fashion, for example as described in European Patent No. EP 0 168 505, incorporated herein by reference.

Analgesic properties of compounds according to Formula (I), such as rotigotine and the like, can, for example, be demonstrated in following two validated animal models.

### Formalin Pain Model:

The mouse formalin test is a chemically-induced sustained pain model with biphasic changes of nociceptive behavior. In mice, the test measures duration of hind paw licking following subplantar injection of formalin. Formalin produces a characteristic biphasic pain response. The early phase reflects the acute pain and the late phase the chronic pain in which spinal/supraspinal plasticity of nociception is considered as a molecular basis. These features have resulted in the formalin test being accepted as a valid model of persistent clinical pain like neuropathic, nociceptive and inflammatory pain (see for example Hunskaar, S., et al., J. Neuroscience Meth 14:69-76, (1985)).

### Tumor Necrosis Factor-Alpha (TNF) Model of Muscular Mechanical Hyperalgesia:

Intramuscular injection of tumor necrosis factor-alpha (TNF) is used as a model of muscular mechanical hyperalgesia, which occurs in the human fibromyalgia, myofascial pain or back pain.

Intramuscular injection of TNF induces mechanical muscle hyperalgesia in rats. This is quantified by measuring the withdrawal threshold to muscle pressure and the grip strength. Mechanical withdrawal thresholds to muscle pressure is measured with an analgesimeter exerting pressure on the gastrocnemius muscle previously injected with TNF. Forelimb grip strength is measured with a digital grip force meter after TNF injection into the biceps brachii muscles. TNF injections do not lead to morphological damage of the muscle (Schäfers et al., Pain 104(3):579-588, (2003)).

Pain on palpation of muscles without morphological abnormalities is typical for fibromyalgia, myofascial pain or back pain in humans (Pongratz DE, et al., Z. Rheumatol 57 Suppl. 2:47-51, (1998)). Thus, the model of intramuscular injection of TNF is used as a model of muscle pain related to fibromyalgia, myofascial pain or back pain. In this model the antinociceptive action of a test compound can be determined, by comparison with a control drug, for example a non-opioid analgesic such as for example metamizol or an anticonvulsants such as pregabalin or gabapentin.

Surprisingly, the compounds to be used according to this disclosure (the compounds of Formula (I)), such as rotigotine and the like, show to have analgesic properties making them suitable for administration to a subject for treatment (including prevention and/or alleviation) of chronic and/or acute pain, in particular non-inflammatory musculoskeletal pain such as back pain, fibromyalgia, and myofascial pain, and in particular in reducing the associated muscular hyperalgesia or muscular allodynia. In particular, the compounds according to Formula (I), more particularly rotigotine, are used for the preparation of a pharmaceutical composition for the prevention, alleviation and/or treatment of fibromyalgia.

Non-limiting examples of types of pain that can be treated by the compounds of the present disclosure are chronic conditions such as musculoskeletal pain, including back pain, fibromyalgia, myofascial pain, pain during menstruation, pain during osteoarthritis, pain during rheumatoid arthritis, pain during gastrointestinal inflammation, pain during inflammation of the heart muscle, pain during multiple sclerosis, pain during neuritis, pain during AIDS, pain during chemotherapy, tumor pain, headache, CPS (chronic pain syndrome), central pain, neuropathic pain such as trigeminal neuralgia, shingles, Stump pain, phantom limb pain, temporomandibular joint disorder, nerve injury, post-herpetic neuralgia, neuropathic pain encountered as a consequence of injuries, amputation infections, metabolic disorders , neuropathic pain associated with diabetes, pseudesthesia, hypothyroidism, uremia, vitamin deficiency or alcoholism; and acute pain such as pain after injuries, postoperative pain, pain during acute gout or pain during operations, such as jaw surgery.

In a particular embodiment, a compound of Formula (I), for example rotigotine is administered for treatment of non-inflammatory musculoskeletal pain such as back pain, fibromyalgia (*e.g.*, in FMS), and myofascial pain (*e.g.*, in MPS), and in particular for reducing muscular hyperalgesia or muscular allodynia associated with such conditions. In a more particular embodiment, the condition to be treated is fibromyalgia.

In another embodiment a compound of Formula (I), for example rotigotine, is administered for treatment of neuropathic pain.

Unless the context demands otherwise, the term "treat", "treating" or "treatment" herein includes preventive or prophylactic use of an agent, for example a compound of Formula (I), in a subject at risk of, or having a prognosis including, pain, or having a condition or syndrome such as FMS or MPS characterized by recurrent pain, as well as use of such an agent in a subject already experiencing pain, as a therapy to alleviate, relieve, reduce intensity of or eliminate such pain or an underlying cause thereof. The standard of care in treating chronic pain is to administer an analgesic agent in anticipation of recurrence of pain, as opposed to allowing the pain to recur before giving further treatment. See for example Grahame-Smith & Aronson, eds., Oxford Textbook of Clinical Pharmacology and Drug Therapy, 2nd ed. Oxford University Press (1992), p. 460.

The term "subject" refers to a warm-blooded animal, generally a mammal such as, for example, a cat, dog, horse, cow, pig, mouse, rat, or primate, including human. In one embodiment the subject is a human, for example a patient having or at risk of a pain condition such as fibromyalgia, myofascial pain or back pain.

The term "central pain" refers to pain associated with a lesion of the central nervous system.

In one embodiment, the compound, for example rotigotine, is administered to a subject suffering from pain, for example on of the types of pain mentioned above, such as fibromyalgia, myofascial pain or back pain, in an analgesic effective amount. The term "effective amount" as used herein means an amount of a compound effective to result in a clinically determinable improvement in, or suppression of, symptoms associated with a medical condition. An improvement in such symptoms can include, in the case of pain symptoms, reduction in intensity, reduction in frequency, or complete cessation of pain for a sustained period of time. An analgesic effective amount for such a subject is equivalent to a therapeutically effective amount as described herein.

There are many methods of application available for administering substituted 2-aminotetralines of Formula (I), in particular rotigotine, which the person skilled in the art can select and adapt depending on the need, condition and age of the subject, the required dosage and the desired application interval. A nonlimiting mode of administration is parenteral, transdermal or mucosal.

In a particular embodiment, the route of administering a substituted 2-aminotetralin of Formula (I), for example rotigotine, is transdermal administration. The form and pharmaceutical composition in which the compound is administered is adapted for the route of administration, and, in the case of transdermal administration a suitable composition can be, for example, an ointment, a gel, a cream, a paste, a spray, a film, a plaster, a patch, a poultice, a cataplasm or an iontophoretic device.

Illustratively, a substituted 2-aminotetralines of Formula (I), for example rotigotine, may be administered by application to a patient's skin of a patch or plaster having the active substance present in an adhesive polymer matrix, for instance a self-adhesive polysiloxane. Examples of suitable transdermal formulations can be found in WO 99/49852, WO 02/ 89777 and WO 02/89778. For the purpose of this invention, these documents are incorporated by reference. Such a form of administration enables a substantially constant plasma level to be established and therefore a constant dopaminergic stimulation over the entire application interval (WO 02/89778, Metman, Clinical. Neurophamacol. 24:163, (2001)). Further, constant delivery by transdermal administration can result in a rapid achievement of a desired dose, particularly by comparison with pulsatile administration of a compound.

If, on the other hand, administration in the form of a subcutaneous or intramuscular depot is desired, a substituted 2-aminotetralines of Formula (I), for example rotigotine, may be suspended, for example as salt crystals, such as crystalline rotigotine hydrochloride, in a hydrophobic anhydrous medium, and injected, such as described in WO 02/15903, incorporated herein by reference, or else administered in the form of microcapsules, microparticles or implants based on biodegradable polymers, such as described, for example, in WO02/38646, incorporated herein by reference..

Other dosage forms suitable for administering a susbstituted 2-amminotetralin of Formula (I), for example rotigotine, are transmucosal formulations, for example sublingual sprays, rectal formulations or aerosols for pulmonary administration.

Suitable dosages of substituted 2-aminotetralines of Formula (I), in particular rotigotine, are typically about 0.05 to about 50 mg/day, for example about 0.1 to about 40 mg/day or about 0.2 to about 20 mg/day, preferably about 4 to about 20 mg/day. Optionally, gradually increasing dosages can be administered, *i.e.* treatment can optionally start with low doses which are incrementally increased until the maintenance dose is reached.

It is clear to the person skilled in the art that the dosage interval may vary depending on the applied quantity, the mode of application and the daily requirement of the patient or subject. Thus, a transdermal form of application may be designed, *e.g.*, for administration once a day, once every three days or once every seven days, whilst a subcutaneous or intramuscular depot can make it possible to administer injections, *e.g.*, in once weekly, bi-weekly or monthly cycles.

The term "transdermal therapeutic system", or its abbreviation "TTS", as used herein refers to a pharmaceutical composition, in a form of one to a plurality of patch or plaster formulations, that contains an active agent, for example a compound of Formula (I) such as rotigotine, and that when applied to skin of a subject delivers at least a portion of the active agent into and across the skin, where the active agent accesses the circulatory system of the subject. A TTS useful herein can be prepared by processes known in the art, for example as described in the publications individually listed below and incorporated herein by reference: U.S. Patent No. 6,562,363 to Mantelle et al., U.S. Patent No. 6,884,434 to Müller & Peck., U.S. Patent Application Publication No. 2003/0026830 of Lauterbach et al., U.S. Patent Application Publication No. 2003/0027793 of Lauterbach et al., U.S. Patent Application Publication No. 2004/0081683 of Schacht et al., U.S. Patent Application Publication No. 2005/0019385 of Houze., U.S. Patent Application Publication No. 2005/0079206 of Schacht et al., U.S. Patent Application Publication No. 2006/0216336 of Wolff.

A TTS useful herein is illustratively of a reservoir or matrix type comprising one or more layers. Typically the TTS has on one side a backing layer and on the opposing side a liner layer that can be removed to expose an adhesive surface or layer that in use contacts the skin surface. The active agent can be distributed, for example as a solution or dispersion, in a matrix formed by the adhesive layer, or it can be present in a separate reservoir layer. The following description of an illustrative matrix-type TTS refers specifically to rotigotine as the active agent but it will be understood that a different compound of Formula (I) or an enantiomer, mixture of enantiomers, pharmaceutically acceptable salt, prodrug or metabolite thereof can be substituted if desired. Such a TTS can consist of one to a plurality of patches of similar composition.

In one embodiment of the invention, a matrix-type TTS for administering rotigotine comprises three layers:
(1) a flexible backing sheet or layer, for example comprising an aluminized polyester foil siliconized on its inner side and coated with a pigment layer or transparent polyester film on its outer side;
(2) a matrix layer that is typically self-adhesive and contains rotigotine distributed therein; a suitable matrix layer comprises an adhesive component, e.g., comprising one or more silicone adhesives, and optionally a compatibilizing component, e.g., comprising a polymer such as povidone, a vinylpyrrolidone/vinyl acetate copolymer or an ethylene/vinyl acetate copolymer, that provides for increased concentration, homogeneity and/or stability of dispersion of the active agent in the matrix layer and/or for enhanced cohesion of the matrix layer; and
(3) a removable liner layer, for example comprising a fluoropolymer-coated polyester film.

The backing and liner layers should be inert to the components of the matrix layer.

The rotigotine can be present in free base or salt (e.g., hydrochloride salt) form or both, but where, as in the present illustrative example, the adhesive matrix is silicone-based it will be found preferable to use rotigotine that is substantially all, for example at least about 95 mol %, at least about 98 mol % or at least about 99 mol %, in free base form.

The matrix layer can be of any suitable thickness but typically is relatively thin, having a total weight of about 10 to about 100 g/m², for example about 20 to about 80 g/m² or about 40 to about 60 g/m². Rotigotine is present in the matrix layer at a concentration illustratively of about 5% to about 25%, for example about 6% to about 20%, about 7% to about 15% or about 8% to about 10%, by weight. In a preferred embodiment, a matrix layer having a total weight of about 50 g/m² (i.e., about 5 mg/cm²) contains rotigotine free base at a concentration of about 9% by weight.

Illustratively rotigotine is present in the TTS in an amount of about 0.05 to about 2.5 mg/cm², for example about 0.1 to about 2 mg/cm², about 0.2 to about 1.5 mg/cm², about 0.3 to about 1 mg/cm² or about 0.4 to about 0.5 mg/cm². In a preferred embodiment, rotigotine free base is present in an amount of about 0.45 mg/cm².

It will be evident that the rotigotine dose present in a TTS can be adjusted by modifying any one or more of matrix weight, rotigotine concentration in the matrix and/or surface area of the TTS. "Surface area" herein refers to the total area of one to a plurality of patches applied at one time to skin of a subject, more specifically to the area of the adhesive matrix in contact with the skin. In one embodiment a series of patches are provided having substantially similar matrix composition, weight and rotigotine concentration, but differing in surface area so as to provide a range of rotigotine dosages.

Typically, a TTS useful herein contains in total about 4 to about 20 mg rotigotine free base. Illustratively, a TTS having a surface area of about 10 cm² contains about 4.5 mg rotigotine free base; a TTS having a surface area of about 20 cm² contains about 9 mg rotigotine free base; a TTS having a surface area of about 30 cm² contains about 13.5 mg rotigotine free base; and a TTS having a surface area of about 40 cm² contains about 18 mg rotigotine free base.

In a silicone-based adhesive matrix, rotigotine free base can be present in solution up to the limit of its solubility in the matrix, but is typically also present in discrete microparticles distributed throughout the matrix. These microparticles can be of any suitable size but it is generally desirable that they be small enough to provide a substantially clear, rather than cloudy or milky, matrix layer. It is also generally desirable that the microparticles comprise rotigotine free base in an amorphous form, to avoid problems that can arise through crystal growth. Use of a compatibilizing agent such as povidone can provide improved physical stability of the matrix layer, for example by inhibiting crystallization of rotigotine. It is believed, without being bound by theory, that in a TTS having povidone in the matrix layer the microparticles comprise a stable amorphous povidone/rotigotine free base complex and act as microreservoirs of rotigotine within the matrix. In a preferred embodiment of the invention, Povidone is present in the matrix layer in a concentration of about 1.5% to about 5% by weight.

One or more silicone adhesives can be used in the matrix layer. Amine-resistant silicone adhesives are preferred. Suitable silicone adhesives include without limitation high-tack silicone adhesives such as BIO-PSA® Q7-4301 of Dow Corning and medium-tack silicone adhesives such as BIO-PSA® Q7-4201 of Dow Coming. In one embodiment both a high-tack and a medium-tack silicone adhesive are present, for example in a weight ratio of about 40:60 to about 60:40, illustratively about 50:50.

Other ingredients are optionally present in the matrix layer, including for example one or more antioxidants and/or antimicrobial preservatives.

A preferred embodiment of the invention relates to a 10 cm² rotigotine patch comprising a matrix layer having the following composition:

Rotigotine free base 4.50 mg, povidone 1.00 mg, BIO-PSA® Q7-4301 22.24 mg, BIO-PSA® Q7-4201 22.23 mg, ascorbyl palmitate 0.01 mg, DL-α-tocopherol 0.025 mg, sodium metabisulfite 0.00045 mg.

Application of one such patch provides an applied dose of 4.5 mg. Application of two, three or four such patches provides an applied dose of 9, 13.5 or 18 mg respectively.

Another preferred embodiment of the invention relates to a 20 cm² rotigotine patch comprising a matrix layer having the following composition:

Rotigotine free base 9.00 mg, povidone 2.00 mg, BIO-PSA® Q7-4301 44.47 mg, BIO-PSA® Q7-4201 44.46 mg, ascorbyl palmitate 0.02 mg, DL-α-tocopherol 0.05 mg, sodium metabisulfite 0.0009 mg.

Application of one such patch provides an applied dose of 9 mg. Application of two such patches provides an applied dose of 18 mg.

Another preferred embodiment of the invention relates to a 30 cm² rotigotine patch comprising a matrix layer having the following composition:

Rotigotine free base 13.50 mg, povidone 3.00 mg, BIO-PSA® Q7-4301 66.71 mg, BIO-PSA® Q7-4201 66.70 mg, ascorbyl palmitate 0.03 mg, DL-α-tocopherol 0.075 mg, sodium metabisulfite 0.00135 mg.

Application of one such patch provides an applied dose of 13.5 mg.

In each case a suitable film for the backing layer is Scotchpak® 1109.

A TTS as described above is suitable for release of rotigotine over a period of about 24 hours, but TTSs with longer or shorter release periods can be used. A TTS having a 24-hour release period as described above is suitable for administration of a daily applied dose of rotigotine of about 0.9 to about 27 mg, more preferably about 4 to about 20 mg. An "applied dose" herein is the amount of rotigotine present in a TTS (whether consisting of one or a plurality of patches) administered to a subject in a day. As is generally the case with transdermal systems, not all of the active agent is released from the TTS and delivered to the subject. Illustratively, if the dose actually received by the subject is about 44% of the applied dose, a 4.5 mg, 9 mg, 13.5 mg or 18 mg applied dose is equivalent respectively to a 2 mg, 4 mg, 6 mg or 8 mg received dose.

In various embodiments, a TTS applied to skin of the subject can be removed after the release period and a further TTS applied at a suitable administration interval, for example about twice daily to about once monthly, or about once daily to about once weekly. Most typically, the TTS is replaced at an interval of about 24 to about 48 hours.

It is not necessary for the TTS to be applied to an area of the subject's body where the sensation of pain occurs. Any skin surface generally suitable for transdermal drug administration can be used as a locus for application of the TTS, including without limitation the front of the abdomen, thigh, hip, flank, shoulder or upper arm. Successive applications of a TTS can be to the same area of skin or to different areas of skin. It can be advantageous to select a different locus on successive days, for example the right side one day and the left side the next day, the upper body one day and the lower body the next day, etc. By varying or rotating the locus of application of the TTS, it will generally be possible to minimize skin irritation or other local reactions to the TTS.

In a preferred embodiment of the invention, rotigotine is administered according to a method of the present invention by applying to skin of the subject (a) a reference TTS having a matrix layer that consists essentially of 4.5 mg rotigotine free base, 1.0 mg povidone, 22.24 mg BIO-PSA® Q7-4301 or a silicone adhesive substantially identical thereto, 22.23 mg BIO-PSA® Q7 4201 or a silicone adhesive substantially identical thereto, 0.01 mg ascorbyl palmitate, 0.025 mg DL-α-tocopherol and 0.00045 mg sodium metabisulfite per 10 cm², and having a total surface area for release of rotigotine of about 10 to about 40 cm², or (b) a TTS that is substantially bioequivalent to the reference TTS. A "substantially bioequivalent" TTS in the present context is one that exhibits, upon administration to human subjects in accordance with standard pharmacokinetic (PK) principles, a bioavailability (as measured, for example, by PK parameters including Cₘₐₓ and AUC₀₋₂₄) that is about 80% to about 125% of that exhibited by the reference TTS. PK data for a reference TTS as defined above may be determined by comparative testing in a PK study, or may be found, for example, in above-referenced U.S. Patent Application Publication No. 2006/0216336.

A substituted 2-aminotetralin compound of Formula (I), for example rotigotine, can be used alone or in a pharmaceutical composition together with a pharmaceutically acceptable carrier.

In one embodiment, the present disclosure comprises the use of a compound of Formula (I), for example rotigotine, in combination with a further active agent for administration to a subject, *e.g.*, a human being in need thereof. The further active agent can be one effective for treatment (including prevention and/or alleviation) of chronic and/or acute pain, in particular for systemic treatment of non-inflammatory musculoskeletal pain, including specific manifestations thereof such as muscular hyperalgesia and/or allodynia, occurring in fibromyalgia, myofascial pain or back pain. The compound of Formula (I), for example rotigotine, and the further active agent may be administered together, *i.e.* in a single dosage form, or may be administered separately, *i.e*. in a separate dose form. If administered separately, the compound of Formula (I), for example rotigotine, and the further active agent can be administered at the same or different times. A therapeutic combination comprising a compound of Formula (I), for example rotigotine, and a further active agent as defined herein is a further embodiment of the present invention.

In a particular embodiment, a pharmaceutical composition is provided comprising a compound of Formula (I), for example rotigotine, and a further active agent effective for treatment (including prevention and/or alleviation) of chronic and/or acute pain, in particular systemic treatment of non-inflammatory musculoskeletal pain, including specific manifestations of thereof such as muscular hyperalgesia and/or allodynia occurring in fibromyalgia, myofascial pain or back pain.

The "further active agent" mentioned above can for example be another analgesic compound such as an opioid, for example fentanyl; a calcitonin gene-related peptide (CGRP) antagonist; for example olcegepant; an N-methyl-D-aspartate (NMDA) receptor blocker, for example dextromethorphan; a cannabinoid; a Cox-2 selective inhibitor; a bradykinin antagonist; acetaminophen; or a NSAID. In other embodiments, the "further active agent" is for example a sedative, antidepressant, tranquillizer, neuroprotective agent, etc.

Nonlimiting examples of opioid and non-opioid analgesics that can be useful in the further active agent include acetaminophen, alfentanil, allylprodine, alphaprodine, anileridine, benzylmorphine, bezitramide, buprenorphine, butorphanol, clonitazene, codeine, cyclazocine, desomorphine, dextromoramide, dextropropoxyphene, dezocine, diampromide, diamorphone, dihydrocodeine, dihydromorphine, dimenoxadol, dimepheptanol, dimethylthiambutene, dioxaphetyl butyrate, dipipanone, dipyrone (metamizol), eptazocine, ethoheptazine, ethylmethylthiambutene, ethylmorphine, etonitazene, fentanyl, heroin, hydrocodone, hydromorphone, hydroxypethidine, isomethadone, ketobemidone, levallorphan, levorphanol, levophenacylmorphan, lofentanil, meperidine, meptazinol, metazocine, methadone, metopon, morphine, myrophine, nalbuphine, nalorphine, narceine, nicomorphine, norlevorphanol, normethadone, normorphine, norpipanone, opium, oxycodone, oxymorphone, papaveretum, pentazocine, phenadoxone, phenazocine, phenomorphan, phenoperidine, piminodine, piritramide, proheptazine, promedol, properidine, propiram, propoxyphene, sufentanil, tilidine, tramadol, NO-naproxen, NCX-701, ALGRX-4975, pharmaceutically acceptable salts thereof, and combinations thereof.

Nonlimiting examples of NSAIDs that can be useful in the further active agent include salicylic acid derivatives (such as salicylic acid, acetylsalicylic acid, methyl salicylate, diflunisal, olsalazine, salsalate and sulfasalazine), indole and indene acetic acids (such as indomethacin, etodolac and sulindac), fenamates (such as etofenamic, meclofenamic, mefenamic, flufenamic, niflumic and tolfenamic acids), heteroaryl acetic acids (such as acemetacin, alclofenac, clidanac, diclofenac, fenchlofenac, fentiazac, furofenac, ibufenac, isoxepac, ketorolac, oxipinac, tiopinac, tolmetin, zidometacin and zomepirac), aryl acetic acid and propionic acid derivatives (such as alminoprofen, benoxaprofen, bucloxic acid, carprofen, fenbufen, fenoprofen, fluprofen, flurbiprofen, ibuprofen, indoprofen, ketoprofen, miroprofen, naproxen, oxaprozin, pirprofen, pranoprofen, suprofen, tiaprofenic acid and tioxaprofen), enolic acids (such as the oxicam derivatives ampiroxicam, cinnoxicam, droxicam, lornoxicam, meloxicam, piroxicam, sudoxicam and tenoxicam, and the pyrazolone derivatives aminopyrine, antipyrine, apazone, dipyrone, oxyphenbutazone and phenylbutazone), alkanones (such as nabumetone), nimesulide, proquazone, MX-1094, licofelone, pharmaceutically acceptable salts thereof, and combinations thereof.

Nonlimiting examples of COX-2 selective inhibitors that can be useful in the further active agent include celecoxib, deracoxib, valdecoxib, parecoxib, rofecoxib, etoricoxib, lumiracoxib, PAC-10549, cimicoxib, GW-406381, LAS-34475, CS-502, pharmaceutically acceptable salts thereof, and combinations thereof.

Nonlimiting examples of NMDA receptor blockers that can be useful in the further active agent include amantadine, D AP5, aptiganel, CPP, dexanabinol, dextromethorphan, dextropropoxyphene, 5,7-dichlorokynurenic acid, gavestinel, ifendopril, ketamine, ketobemidone, licostinel, LY-235959, memantine, methadone, MK 801, phencyclidine, remacemide, selfotel, tiletamine, pharmaceutically acceptable salts thereof, and combinations thereof.

Nonlimiting examples of sedatives that can be useful in the further active agent include without limitation acylic ureides, alcohols, amides, barbituric acid derivatives, benzodiazepine derivatives, bromides, carbamates, chloral derivatives, quinazolone derivatives and piperidinediones. Specific examples include acecarbromal, acetal, acetophenone, aldol, allobarbital, ammonium valerate, amobarbital, aprobarbital, apronalide, barbital, brallobarbital, bromisovalum, bromoform, brotizolam, butabarbital, butalbital, butallylonal, butethal, butoctamide, calcium bromolactobionate, capuride, carbocloral, carbromal, carbubarb, carfimate, chloral betaine, chloral formamide, chloral hydrate, α-chloralose, chlorhexadol, cinolazepam, clomethiazole, cyclobarbital, cyclopentobarbital, cypripedium, dexmedetomidine, dichloralphenazone, diethylbromoacetamide, doxefazepam, doxylamine, ectylurea, enallylpropymal, estazolam, etaqualone, ethchlorvynol, ethinamate, etodroxizine, etomidate, febarbamate, flunitrazepam, flurazepam, glutethimide, haloxazolam, heptabarbital, hexapropymate, hexethal, hexobarbital, hydrobromic acid, isovaleryl diethylamide, loprazolam, lormetazepam, mecloqualone, menthyl valerate, meparfynol, mephobarbital, methaqualone, methitural, methyprylon, midazolam, narcobarbital, nealbarbital, niaprazine, nimetazepam, nitrazepam, opium, paraldehyde, pentaerythritol chloral, pentobarbital, tert-pentyl alcohol, perlapine, phenallymal, phenobarbital, phenylmethylbarbituric acid, piperidione, propallylonal, propiomazine, proxibarbal, pyrithyldione, quazepam, reposal, rilmazafone, secobarbital, sulfonethylmethane, sulfonmethane, talbutal, temazepam, tetrabarbital, thalidomide, triazolam, 2,2,2-trichloroethanol, triclofos, trimetozine, valdetamide, vinbarbital, vinylbital, zaleplon, zolpidem, zopiclone, pharmaceutically acceptable salts thereof, and combinations thereof.

Nonlimiting examples of tranquilizers that can be useful in the further active agent include without limitation anxiolytics such as arylpiperazines, benzodiazepine derivatives and carbamates. Specific examples include abecarnil, alpidem, alprazolam, benzoctamine, bromazepam, buspirone, camazepam, captodiamine, chlordiazepoxide, chlormezanone, clobazam, clorazepic acid, clotiazepam, cloxazolam, diazepam, emylcamate, enciprazine, ethyl loflazepate, etifoxine, etizolam, flesinoxan, fludiazepam, fluoresone, flutazolam, flutoprazepam, glutamic acid, halazepam, hydroxyphenamate, hydroxyzine, ipsapirone, ketazolam, lesopitron, lorazepam, loxapine, medazepam, meprobamate, metaclazepam, mexazolam, nordazepam, oxazepam, oxazolam, pazinaclone, pinazepam, prazepam, suriclone, tandospirone, tofisopam, tybamate, valnoctamide, pharmaceutically acceptable salts thereof, and combinations thereof.

Nonlimiting examples of antidepressants that can be useful in the further active agent include without limitation bicyclic, tricyclic and tetracyclic antidepressants, hydrazides, hydrazines, phenyloxazolidinones and pyrrolidones. Specific examples include adinazolam, adrafinil, amineptine, amitriptyline, amitriptylinoxide, amoxapine, befloxatone, bupropion, butacetin, butriptyline, caroxazone, citalopram, clomipramine, cotinine, demexiptiline, desipramine, dibenzepin, dimetacrine, dimethazan, dioxadrol, dothiepin, doxepin, duloxetine, etoperidone, femoxetine, fencamine, fenpentadiol, fluacizine, fluoxetine, fluvoxamine, hematoporphyrin, hypericin, imipramine, imipramine N-oxide, indalpine, indeloxazine, iprindole, iproclozide, iproniazid, isocarboxazid, levophacetoperane, lofepramine, maprotiline, medifoxamine, melitracen, metapramine, metralindole, mianserin, milnacipran, minaprine, mirtazapine, moclobemide, nefazodone, nefopam, nialamide, nomifensine, nortriptyline, noxiptilin, octamoxin, opipramol, oxaflozane, oxitriptan, oxypertine, paroxetine, phenelzine, piberaline, pizotyline, prolintane, propizepine, protriptyline, pyrisuccideanol, quinupramine, reboxetine, ritanserin, roxindole, rubidium chloride, sertraline, sulpiride, tandospirone, thiazesim, thozalinone, tianeptine, tofenacin, toloxatone, tranylcypromine, trazodone, trimipramine, tryptophan, venlafaxine, viloxazine, zimeldine, pharmaceutically acceptable salts thereof, and combinations thereof.

Nonlimiting examples of neuroprotective agents that can be useful in the further active agent include aptiganel, citicoline, dexanabinol, ebselen, licostinel, lubeluzole, remacemide, repinotan, riluzole, xaliproden, ziconotide, pharmaceutically acceptable salts thereof, and combinations thereof.

In a particular embodiment, a compound of Formula (I), for example rotigotine, is administered in combination with dextromethorphan.

Combination therapy can involve, for example, simultaneous or sequential delivery of the two active agents. Sequential administration can be achieved using a single dosage form, for example a dosage form such as an oral tablet that has two different layers with different release profiles for the two active ingredients. One of ordinary skill in the art will appreciate that various other forms of administration and application patterns are conceivable within the context of the present disclosure, all of which form subject matter of the invention.

Rotigotine and other compounds of this disclosure are structurally different from dopamine agonists such as pramipexole and ropinirole previously disclosed to be useful for treatment of pain.

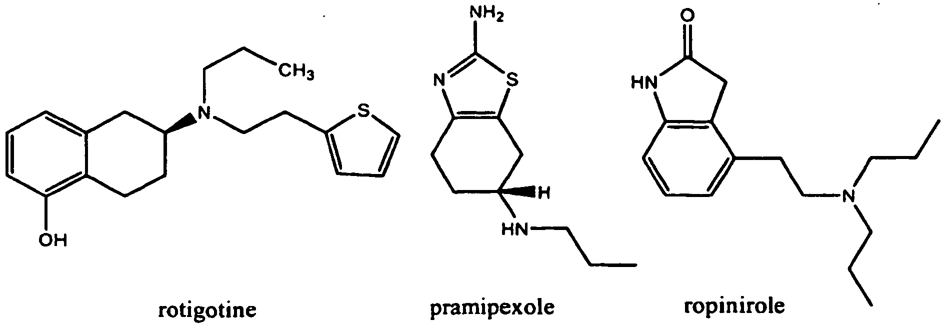

Rotigotine, for instance, is a non-ergolinic dopamine agonist binding to all dopamine receptors with a clear preference for the D3 receptor. It has greater affinity for the D1 receptor than pramipexole and ropinirole and is also an agonist of the 5-HT_{1A} receptor and an antagonist of the α2B receptor. It is believed, without being bound by theory, that the affinity of rotigotine for the 5-HT_{1A} receptor is of particular significance, as dysfunction in serotonin (5-HT) and norepinephrine transmission may influence pain in patients with fibromyalgia. See, for example, Littlejohn et al., Current Pharmaceutical Design 12:3-9 (2006).

Compounds of Formula (I), for example rotigotine, may provide a lower likelihood of augmentation and rebound effects in comparison to other dopaminergic agents, such as for example levodopa. In a recent restless leg syndrome (RLS) study, a number of patients receiving long-term treatment with pramipexole experienced augmentation effects. See Happe et al., CNS Drugs 18(1):27-36 (2004). Augmentation effects include intensification of symptoms following long term use of a compound. Rebound effects include increased occurrence of symptoms as the compound dosage wears off.

As shown in the following examples, rotigotine, an illustrative substituted 2-aminotetralin compound of Formula (I), shows a dosis-dependent antinociceptive effect in an animal model of non-inflammatory musculoskeletal pain.

The words "comprise", "comprises", and "comprising" as used herein are to be interpreted inclusively rather than exclusively.

### Figure Legends

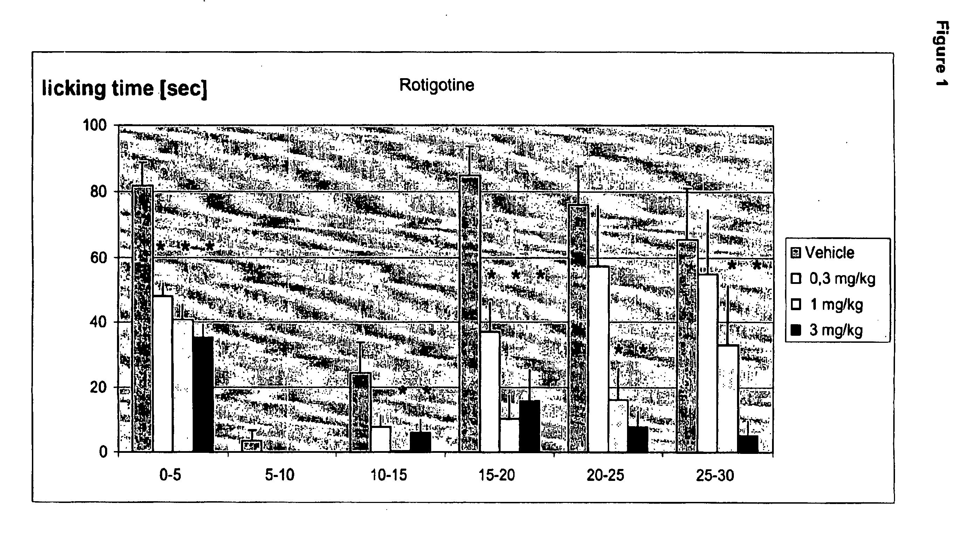

- Figure 1:: Dose-dependent effect (0.3 mg/kg, 1 mg/kg, 3 mg/kg) of rotigotine on time course of formalin-induced licking. Y-axis shows licking time in seconds (sec). X-axis displays the time in minutes (min). A star (·) indicates a significant difference from vehicle (ANOVA corrected for multiple comparisons (P< 0.05).

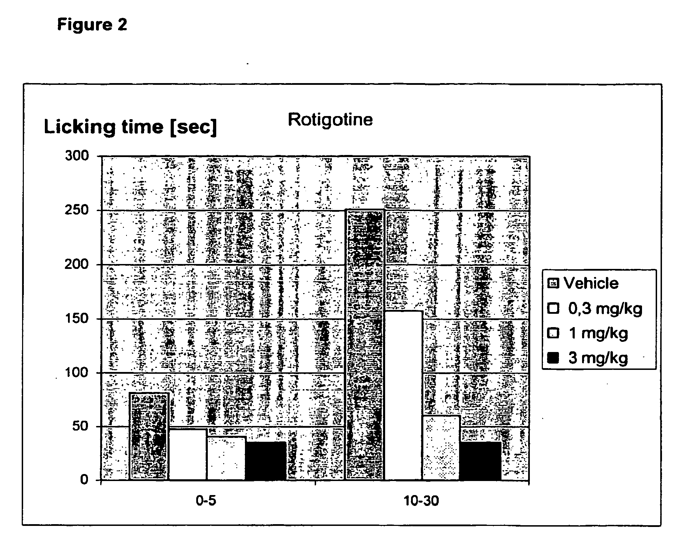

- Figure 2:: Dose-dependent effect (0.3 mg/kg, 1 mg/kg, 3 mg/kg) of rotigotine on two phases of formalin-induced licking. Y-axis shows licking time in seconds (sec). X-axis displays the time in minutes (min).
- Figure 3:: % Maximal Possible Effect (MPE) of Rotigotine and Metamizol on Withdrawal Pressure. * p < 0.05 (ANOVA + Bonferroni post-hoc) - versus PBS, MW +/- SEM. + p < 0.05 (Mann-Whitney-U-test) - versus PBS, MW +/- SEM.
- Figure 4:: % MPE of Rotigotine and Metamizol on Grip Force. ++ p < 0.01 (Mann-Whitney-U-test) - versus PBS, MW +/- SEM.

### Examples

### Example 1: Formalin Pain Model

Rotigotine (SPM 962 base) was evaluated for possible analgesic activity in the mouse formalin test in which hind paw licking time was measured at 5-minute intervals for 30 minutes following subplantar injection of formalin.

Rotigotine was administered intraperitoneally to 10 CD-1 (*Crl.*) derived mice weighing 22 ± 2 g (provided by BioLasco Taiwan under Charles River Laboratories Technology Licensee). Rotigotine (3, 1, and 0.3 mg/kg) in a vehicle (5 ml/kg) comprising 0.2% hydroxypropylmethycellulose (HPMC) and 0.9% NaCl, and a vehicle (5ml/kg) alone as a control, were each administered by intraperitoneal injection 30 minutes before suplantar injection of formalin (0.02 ml, 2% solution). Reduction of the formalin-induced hind paw licking time was recorded at 5-minute interval during the following 0 to 30 minute period after formalin injection. A recuction of licking time of 50 percent or more (≥50%) indicates significant analgesic and anti-phlogistic activity. Statistical analysis was performed by using one-way ANOVA (Analysis for Variance) and Dunnett's test to compare rotigotine-treated and vehicle control groups. Significance is considered at P< 0.05 level. Observation of animals for acute toxic symptoms and autonomic effects was performed before formalin injection.

Results are summarized in Table 1. Rotigotine exhibited significant dose-dependent analgesic activity in early and late phase. Significant reduction in formalin-induced hind paw licking time was observed over the vehicle control with rotigotine at all three doses at least at the 0 to 5 and 15 to 20 minute intervals. Significant reduction in hind paw licking time ws observed with the 1 mg/kg and 3 mg/kg rotigotine-treated groups at the 10 to 15, 20 to 25 and 25 to 30 minute intervals. No significant central or autonomic signs were observed.

**Table 1: Results of the Formalin-Test**

| **Treatment** | **Hind paw licking time (seconds)** | | | | | | |
|---|---|---|---|---|---|---|---|
| | **Time (min)** | **0-5** | **5-10** | **10-15** | **15-20** | **20-25** | **25-30** |
| Rotigotine 3 mg/kg | Average | 35.6 | 0 | 6 | 15.9 | 7.8 | 5.5 |
| | SEM | 3.9 | 0 | 4.1 | 10 | 4.8 | 4.3 |
| | % Inhibition | (56) | (100) | (76) | (81) | (90) | (92) |
| Rotigotine 1 mg/kg | Average | 41.0 | 0 | 0.2 | 10.3 | 16.3 | 33.5 |
| | SEM | 4.6 | 0 | 0.2 | 7.1 | 9.9 | 17.9 |
| | % Inhibition | (50) | (100) | (99) | (88) | (79) | (49) |
| Rotigotine 0.3 mg/kg | Average | 47.9 | 0.0 | 8.0 | 37.3 | 57.2 | 54.8 |
| | SEM | 4.1 | 0.0 | 3.3 | 8.9 | 18.6 | 19.8 |
| | % Inhibition | (41) | (100) | (67) | (56) | (25) | (16) |
| Vehicle | Average | 81.6 | 3.7 | 24.6 | 84.5 | 76.1 | 65.4 |
| | SEM | 6.9 | 3.2 | 9.4 | 9.1 | 11.3 | 15.7 |

| | | | | | | | |
|---|---|---|---|---|---|---|---|
| SEM = standard error of the mean | | | | | | | |

### Example 2: Tumor Necrosis Factor-Alpha (TNF) Model of Muscular Mechanical Hyperalgesia

### Animals, induction of muscle pain

Adult male Sprague Dawley rats (from Charles River Sulzfeld, Germany), with a body weight of 220 g to 300 g were group-housed (3 animals per cage) and maintained in a room with controlled temperature (21-22°C) and a reversed light-dark cycle (12 h/12 h) with food and water available *ad libitum.*

Recombinant rat tumor necrosis factor alpha (TNF) obtained from R&D Systems, Minneapolis, MN, USA was diluted in 0,9% NaCl and used in a concentration of 1 µg in 50 µl. Injections to induce muscle pain were performed on rats under a short halothane narcosis with a 30 g needle bilaterally into the gastrocnemius, or into the biceps brachii muscle. All rats were habituated to the behavioral tests before injections and baseline values were recorded over three test days.

### Behavioral readout: muscle pressure (Randall-Selitto)

Mechanical withdrawal thresholds to muscle pressure were then measured with an analgesimeter (Ugo Basile, Comerio, Italy) according to the Randall-Selitto method. The rats were permitted to crawl into a sock, allowing them to relax. The hind limbs of the rats were positioned such that an increasing pressure was applied onto the gastrocnemius muscle (maximum 250 g). The pressure needed to elicit withdrawal was recorded. Means of 3 trials for each hind limb are calculated (interstimulus interval of >30 sec). ). Three pre-tests were performed on day -3, -2, -1, testing the left and right side in sequel. Pre-test values varied only minimally over these 3 days. The mean of the withdrawal thresholds of each rat for the 3 pre-testing days was determined and taken for analysis. Only animals with a significant TNF effect were included for further analysis.

The rats were injected with TNF into the gastrocnemius muscle. After 18 hours, the rats were tested for pressure hyperalgesia pre-application and 15 to 60 minutes post-applicaton of rotigotine.

### Behavioral readout: grip strength

Grip strength of the rat forelimbs was tested with a digital grip force meter (DFIS series, Chatillon, Greensboro, NC, USA).

Three pre-tests were performed on day -3, -2, -1. Since no relevant training effect for the grip strength testing could be observed, the baseline was calculated as mean of the 3 pre-test measurements and taken for further analysis. The effect of the TNF injection was calculated for each animal separately and only animals with a significant TNF effect were included for further analysis.

Phase 2: Rats were injected with TNF into the biceps brachii muscle. Six hours later, grip strength of the forelimbs was tested with a digital grip force meter. The rats were positioned to grab a grid with the forelimbs and were gently pulled so that the grip strength could be recorded. Means of ten trials were calculated.

### Application protocol

A pilot study was performed to show that injection of 1 µg TNF i.m. into the gastrocnemius muscle is sufficient to induce pressure hyperalgesia. The rats were then placed in groups of 10 and treated with 0.3, 1.0 or 3.0 mg/kg rotigotine or a vehicle intraperitoneally. Injection volume of intraperitoneal injections was 0.5 ml (weight dependent). Grip strength was again tested after 15 to 60 minutes, following injection of rotigotine.

### Data presentation and statistics

Data are shown in graphs displaying means and SEMs. Pre- and post-treatment data were compared using ANOVA (Analysis Of Variance) and a Tukey post hoc test. Means of treatment groups were compared using a one-way ANOVA and Bonferroni's post hoc test or a Mann-Whithney-U test for comparison of Metamizol treatment group versus vehicle. Maximal possible effects (MPE) were calculated for all types of treatment. Only rats in which withdrawal thresholds were significantly reduced after TNF injection were included.

### Results

Withdrawal thresholds to pressure applied percutaneously to muscle are markedly reduced after TNF injection in most rats. This primary muscular hyperalgesia parallels tenderness to palpation that is observed clinically in patients with myalgia, such as myofascial pain, fibromyalgia and back pain. See McCain in Wall and R Melzack (Eds.); Textbook of Pain, New York: Churchill Livingstone (1994) pp. 475-493). Tenderness to palpation is a primary criterion for the diagnosis of muscle pain under clinical and experimental human conditions (Wolfe F, et al., The American College of Rheumatology 1990 criteria fo the classification of fibromyalgia: report of the multicenter criteria committee. Arthritis Rheum 1990, 33:160-172; Arendt-Nielsen L. in TS Jensen, JA Turner and Z Wiesenfeld-Hallin (Eds.), Proceedings of the 8th

World Congress of Pain: IASP Press, Seattle, (1997), pp. 393-425).

Table 2 shows the absolute values of withdrawal thresholds to pressure without injection of TNF. Withdrawal thresholds remained stable after phosphate-buffered saline (PBS) injection. Significant higher withdrawal thresholds was seen with rotigotine 1 mg/kg.

**Table 2: Effect of Rotigotine on Withdrawal Pressure. * p < 0.05**

| **Group** | **Mean (g)** | **SEM** |
|---|---|---|
| Control | 9.1; 8.6; 9.6 | 0.3; 0.3; 0.3 |
| Saline | 9.2; 9.3; 9.7 | 0.3; 0.3; 0.5 |
| Rotigotine, 0.3 mg/kg | 9.1 | 0.4 |
| Rotigotine, 1.0 mg/kg | 10.8* | 0.6 |
| Rotigotine, 3.0 mg/kg | 9.7 | 0.4 |

Table 3 shows the absolute values of withdrawal thresholds to pressure with injection of TNF.

**Table 3: Effect of Rotigotine on Withdrawal Pressure**

| **Group** | **Mean (g)** | **SEM** |
|---|---|---|
| Control | 9.8; 9.6; 9.7; 9.6 | 0.3; 0.3; 0.2; 0.2 |
| TNF | 5.7; 6.0; 6.1; 6.8 | 0.2; 0.2; 0.2; 0.2 |
| PBS | 6.4 | 0.2 |
| Rotigotine, 0.3 mg/kg | 5.6 | 0.2 |
| Rotigotine, 1.0 mg/kg | 6.5 | 0.2 |
| Rotigotine, 3.0 mg/kg | 6.5 | 0.3 |

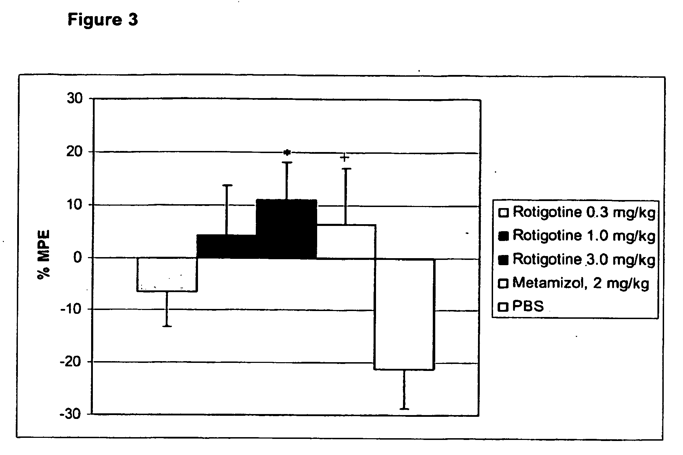

As shown in Table 4 and Fig. 3, the percent of Maximal Possible Effect (% MPE) was significantly different from vehicle for Rotigotine at 3 mg/kg and Metamizol 2mg/kg. Vehicle had no effect.

**Table 4: % MPE of Rotigotine and Metamizol on Withdrawal Pressure (see also Figure 3)**

| * p < 0.05 (ANOVA + Bonferroni post-hoc) - versus PBS, MW +/- SEM. + p < 0.05 (Mann-Whitney-U-test) - versus PBS, MW +/- SEM. | | |
|---|---|---|
| **Group** | **Mean (%)** | **SEM** |
| Rotigotine, 0.3 mg/kg | 6.6 | 6.6 |
| Rotigotine, 1.0 mg/kg | 4.8 | 9.4 |
| Rotiqotine, 3.0 mg/kg | 10.9 (*) | 7.1 |
| Metamizol, 2 mg/kg | 6.2 (+) | 10.7 |
| PBS | -21.3 | 7.5 |

Table 5 shows the absolute values of grip strength without injection of TNF. The grip strength values were all stable after saline injection.

**Table 5: Effect of Rotigotine on Grip Strength (without TNF injection)**

| **Group** | **Mean (N)** | **SEM** |
|---|---|---|
| Control | 8.5; 8.3; 9.1 | 0.2; 0.2; 0.1 |
| Saline | 8.1; 8.1; 9.3 | 0.7; 0.5; 0.3 |
| Rotigotine, 0.3 mg/kg | 7.5 | 0.5 |
| Rotigotine, 1.0 mg/kg | 7.8 | 0.3 |
| Rotigotine, 3.0 mg/kg | 9.0 | 0.1 |

Table 6 shows the absolute values of grip strength after injection of TNF.

**Table 6: Effect of Rotigotine on Grip Strength (with TNF injection)**

| **Group** | **Mean (N)** | **SEM** |
|---|---|---|
| Control | 9.1; 9.1; 8.9; 9.6 | 0.2; 0.1; 0.1; 0.1 |
| TNF | 7.7; 7.0; 7.0; 7.1 | 0.2; 0.4; 0.2; 0.3 |
| PBS | 7.1 | 0.2 |
| Rotigotine, 0.3 mg/kg | 7.8 | 0.3 |
| Rotigotine, 1.0 mg/kg | 7.6 | 0.2 |
| Rotigotine, 3.0 mg/kg | 7.6 | 0.3 |

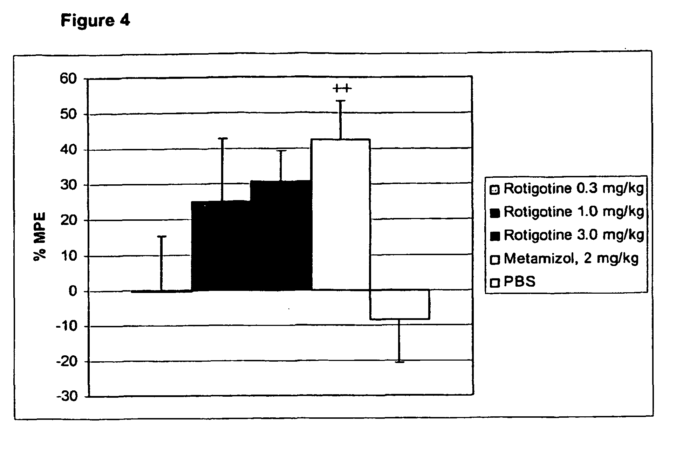

As shown in Table 7 and Fig. 4, the percent of Maximal Possible Effect (% MPE) was significantly different from vehicle for Metamizol 2mg/kg. Vehicle had no effect.

**Table 7: % MPE of Rotigotine and Metamizol on Grip Strength (see also Figure 4)**

| ++ p < 0.01 (Mann-Whitney-U-test) - versus PBS, MW +/- SEM. | | |
|---|---|---|
| **Group** | **Mean (%)** | **SEM** |
| Rotigotine, 0.3 mg/kg | 0.2 | 15.5 |
| Rotigotine, 1.0 mg/kg | 24.9 | 17.9 |
| Rotigotine, 3.0 mg/kg | 30.5 | 8.9 |
| Metamizol, 2 mg/kg | 42.4 (++) | 11.1 |
| PBS | -8.4 | 12.2 |

From the results of this study it can be concluded that rotigotine induces a dosis-dependent reduction of muscular hyperalgesia induced by TNF injected into muscle.

### Example 3: Parallel, Randomized, Double-blinded, Placebo-Controlled Proof of Concept Trial to Assess the Efficacy and Safety of Rotigotine in Subjects with Signs and Symptoms Associated with Fibromyalgia Syndrome

This proof of concept trial investigates the efficacy and safety of 2 doses of rotigotine in adult male and female subjects with fibromyalgia syndrome. This trial is a randomized, double-blind, placebo-controlled, multicenter trial.

The overall post-Baseline duration of treatment is 13 weeks. The trial consists of a 4-week Titration Phase, an 8-week Maintenance Phase, a 1-week De-escalation Phase, and a 2-week Safety Follow-Up Phase. If subjects meet the eligibility criteria, they are randomized to receive either rotigotine 4mg/24hrs, rotigotine 8mg/24hrs, or placebo during the Maintenance Phase. Subjects assigned to rotigotine are titrated at weekly intervals of 2mg/24hrs until they reach 4mg/24hrs or 8mg/24hrs. All subjects completing the 4-week Titration Phase enter an 8-week Maintenance Phase. No dose adjustment is allowed during the Maintenance Phase. The Treatment Phase is defined as the combined Titration and Maintenance Phases.

The primary variable for this trial is the change in average Likert pain score from Baseline to the last 2 weeks of the Treatment Phase. The secondary efficacy variables are the Fibromyalgia Impact Questionnaire (FIQ) total score and associated subscores, the total myalgic score (the numerical assessment of pain from palpation of 18 possible tender points), subject's perception of interference with sleep and general activity, and the Patient Global Impression of Change (PGIC) scale. Other variables include the Beck Depression Inventory-II (BDI-II), Hospital Anxiety and Depression Scale (HADS) depression and anxiety subscale scores, use of rescue medication (including alcohol) for pain, fibromyalgia symptom checklist, presence of impulse control disorders, sleep attacks, menstrual/sexual function, and pharmacokinetic assessments. Subjects use a paper diary in the morning and evening to record pain intensity, pain interference with sleep and general activity, and use of rescue medication.

Approximately 25 sites are selected to meet the recruitment time line. In order to randomize 240 subjects (80 subjects per treatment arm) approximately 480 subjects are enrolled.

Trial Design: Variables to be assessed are the following:

Primary variable: Within-subject change in average daily pain score from

Baseline to the last 2 weeks of the Treatment Phase using an 11-point Likert scale (0-10).

Secondary variables (efficacy): Within-subject change from Baseline to Endpoint in Fibromyalgia Impact Questionnaire (FIQ) (0-100); Within-subject change from Baseline to Endpoint in total myalgic score (0-54); Within-subject change in average daily interference with sleep from Baseline to the last 2 weeks of the Treatment Phase using an 11-point Likert scale (0-10); Within-subject change in average daily interference with general activity from Baseline to the last 2 weeks of the Treatment Phase using an 11-point Likert scale (0-10); Global perception of change in pain from Baseline to Endpoint using the Patient Global Impression of Change scale (PGIC); Within-subject change from Baseline to the last 2 weeks of the Treatment Phase in average morning pain score; Within-subject change from Baseline to the last 2 weeks of the Treatment Phase in average evening pain score.

Secondary variables (other): Within-subject change from Baseline on the Beck Depression Inventory-II (BDI-II); Within-subject change from Baseline on the Hospital Anxiety Depression Scale (HADS) depression and anxiety subscale scores; Use of rescue medication (including alcohol) for pain; Changes in fibromyalgia symptom checklist; Presence of impulse control disorders (assessed by the Jay Modified Minnesota Impulsive Disorders Interview [MIDI]); Plasma concentrations of rotigotine.

Secondary variables (safety): Observation and assessment of adverse events (AEs); Changes in laboratory parameters (including endocrine parameters); Changes in vital sign measurements (blood pressure, pulse, temperature, body weight); Changes in physical examination findings; Changes in 12-lead electrocardiograms (ECGs); Presence of sleep attacks; Changes in menstrual/sexual function; Subject withdrawals due to AEs.

### Trial Description:

The overall trial consists of the Screening Phase through the end of the Safety Follow-Up Phase (see Table 8).

**Table 8: Trial Description**

| Screening (Visit 1) | | |
|---|---|---|
| | Washout of prohibited medications | up to 4 weeks |
| | Baseline Diary Phase | 7 days prior to Baseline (Visit 2)* |
| Baseline (randomization; Visit 2) | | |
| Titration | | 4 weeks |
| Maintenance | | 8 weeks |
| De-escalation | | 1 week |
| Safety Follow-Up | | 2 weeks |

| | | |
|---|---|---|
| * Subjects complete the diary each day beginning at Screening (Visit 1); the 7 days prior to Baseline (Visit 2) are used to determine eligibility for randomization. | | |

### Trial Treatment:

Subjects who complete the Screening Phase enter the Titration Phase at Visit 2 (Baseline) and are randomized to 1 of 3 different treatment groups:Rotigotine 4mg/24hrs, Rotigotine 8mg/24hrs and Placebo.

Two different patch sizes are used (10cm² and 20cm²). Active patches deliver either 2mg/24hrs or 4mg/24hrs of rotigotine. Placebo patches are matched according to size and appearance.

### Methods for Assessing Efficacy Parameters

The efficacy parameters are assessed using - among others - the following rating scales, questionnaires, and assessments:

Likert scales: For the subject's assessment of his/her condition, an 11-point Likert scale is used. Subjects complete the diary daily in the morning and evening as specified. Pain scale - the subject rates his/her average pain over the last 12 hours, from 0 (no pain) to 10 (worst pain ever experienced) (morning and evening diary). Sleep scale - the subject rates quality of sleep, from 0 (very good sleep) to 10 (very poor sleep) (morning diary only), if sleep was sufficient (Yes/No), and if the subject awoke rested (Yes/No). General activity scale - the subject rates how the pain has interfered with general activity over the past 12 hours, from 0 (did not interfere) to 10 (completely interfered) (evening diary only).

Fibromyalgia Impact Questionnaire (FIQ): The FIQ is a self-administered instrument composed of 20 questions. It is completed at the beginning of the visit. The first item contains 11 questions are related to physical functioning; each question is rated on a 4-point Likert-type scale. Questions 12 and 13 ask the subject to mark the number of days he/she felt well and the number of days he/she was unable to work (including housework) because of fibromyalgia symptoms. Questions 14 through 20 are horizontal linear scales marked in 10 increments on which the subject rates work difficulty, pain, fatigue, morning tiredness, stiffness, anxiety, and depression.

Patient Global Impression of Change (PGIC): The PGIC is a 7-point self-administered categorical rating scale in which the subject rates the change in his/her pain since starting trial medication (from much worse [score of 1] to much better [score of 7]).

Total myalgic score: The total myalgic score is based on clinician assessment of the 18 tender points associated with fibromyalgia. The investigator should press on each tender point with enough pressure (4kg/cm²) to have the skin under the thumbnail blanch. Each point is rated on a scale of 0 to 3 (0=no pain, 1=pain is reproduced, 2=focal response to pain, 3=subject flinches or withdraws), and the total score is summed. The maximum myalgic score is 54. Every attempt should be made to have the same clinician perform this assessment for all subjects throughout the trial.

## Claims

1. Use of a compound having the Formula (I) 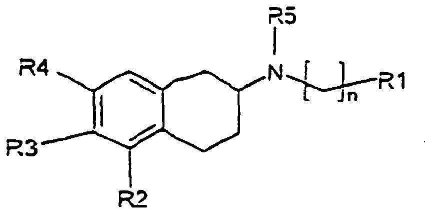 wherein
n is a number from 1 to 5;
R1 is selected from the group consisting of hydrogen, 3-pyridyl, 4-pyridyl, optionally substituted phenyl, 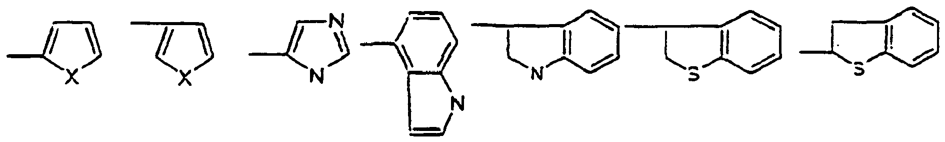 wherein X is S, O or NH:
R2 is a group -OA; and R3 and R4 are each independently hydrogen or a group -OA; wherein A is hydrogen, alkyl, cycloalkyl, aryl, alkoxyalkyl, -C(=S)R6, - C(=S)OR6, C(=S)NR6R7, -S(O)₂R6, -S(O)₂OR6, -P(O₂H)R6, -P(O₂H)OR6, - CHR6OC(O)R7, -C₁₋₃ alkyl-OC(O)R6, -C(OR6)R7R8, -CH(OR6)R7, -C(O)R6, - C(O)NR5R7, or C(O)OR6,
wherein R6, R7 and R8 are each independently hydrogen, alkyl, cycloalkyl, or
aryl; and wherein the alkyl substituents are optionally substituted with one ore more halogen atoms;
R5 is a C₁₋₃ alkyl;
wherein the compounds of Formula (I) can be present as pure enantiomers (R or S) or any mixture thereof, including racermates, or pharmaceutically acceptable salts on a metabolite thereof for the preparation of a pharmaceutical composition for the prevention, alleviation and/or treatment of chronic and/or acute pain, wherein the metabolite is (S)-2-N-propylamino-5-hydroxyketalin, and wherein the pain is selected from musculoske letal pain, including back pain, fibromyalgia, myofascial pain, pain during menstruation, pain during ostecarthritis, pain during rheumatoid arthritis, pain during gastrointestinal inflammation, pain during inflammation of the heart muscle, pain during multiple sclerosis, pain during neuritis, pain during AIDS, pain during chemotherapy, tumor pain, headache, CPS (chronic pain syndrome), central pain, neuropathic pain such as trigeminal neuralgia, shingles, stump pain, phantom limb pain, temporomandibular joint disorder, nerve injury, , post- herpetic neuralgia or neuropathic pain encountered as a consequence of injuries, amputation infections, metabolic disorders , neuropathic pain associated with diabetes, pseudesthesia, hypothyroidism, uremia, vitamin deficiency or alcoholism; and acute pain such as pain after injuries, postoperative pain, pain during acute gout or pain during operations, such as jaw surgery

2. Use of a compound according to claim 1 wherein the pharmaceutical composition is provided for non-inflammatory musculoskeletal pain.

3. Use of a compound according to claim 2, wherein the pharmaceutical composition is provided for fibromyalgia, myofascial pain or back pain.

4. Use of a compound according to claim 3, wherein the pharmaceutical composition is provided for fibromyalgia.

5. Use of a compound according one of the preceding claims, wherein the pharmaceutical composition is provided for reducing muscular hyperalgesis or muscular allodynia.

6. Use according to one of the preceding claims, wherein A is a hydrogen atom or a group selected from 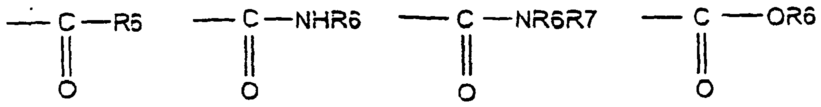 wherein R6 and R7 are each independently C₁₋₁₂ alkyl, phenyl, or methoxyphenyl.

7. Use according to one of the preceding claims, wherein n is 1,2 or 3.

8. Use according to one of the preceding claims, wherein R1 is selected from the group 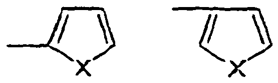 wherein X is S, O or NH.

9. Use according to one of the preceding claims, wherein X is a sulphur atom.

10. Use according to one of the preceding claims, wherein R5 is a C₃ alkyl.

11. Use according to one of the preceding claims, wherein R1 is a 2-thienyl, R3 and R4 are both hydrogen, R5 is a C₃ alkyl and n is 2.

12. Use according to one of the preceding claims, wherein the compound is (+/-) 5,6,7,8-tetrahydro-6-[propyl[2-(2-thienyl)ethyl]amino]-1-naphthol or a pharmaceutical acceptable salt or metabolite thereof, wherein an metabolite is (S)-2-N-propylamino-5-hydroxyhydraline

13. Use according to claim 12, wherein the compound is the pure S-enantiomer (rotigotine) or rotigotine hydrochloride.

14. Use according to one of the preceding claims, wherein the pharmaceutical composition is provided for parental, transdermal or mucosal administration.

15. Use according to one claim 14, wherein the pharmaceutical composition is provided for transdermal administration.

16. Use according to one of the preceding claims, wherein a compound of general formula (I) is administered in a dose of 0.05 to 50 mg per day.

17. Use according to one of the preceding claims, further comprising administering at least one further active agent.

18. Use according to claim 17, wherein the at least one further active agent comprises an opioid, a CGRP antagonist, an NMDA receptor blocker, a cannabinoid, a bradykinin antagonist, acetaminophen, an NSAID, a COX-2 selective inhibitor, a sedative, an antidepressant, a tranquilizer and/or a neuroprotective agent.

19. Use according to claim 17, wherein the the further active compound is dextromethorphan.

## Patentansprüche

1. Verwendung einer Verbindung mit der Formel (I): 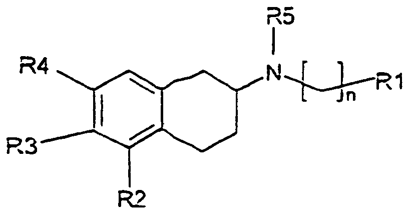 wobei gilt:
n ist eine Zahl von 1 bis 5;
R1 ist ausgewählt aus der Gruppe bestehend aus Wasserstoff, 3-Pyridyl, 4-Pyridyl, gegebenenfalls substituiertes Phenyl, 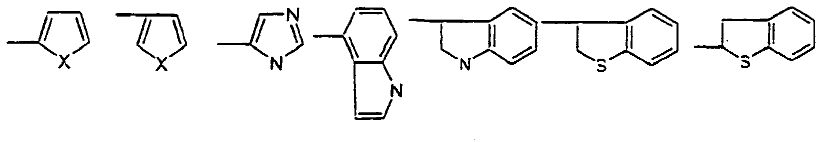
wobei X S, O oder NH ist;
R2 ist eine Gruppe -OA; und R3 und R4 sind jeweils unabhängig Wasserstoff oder eine Gruppe -OA, wobei A Wasserstoff, Alkyl, Cycloalkyl, Aryl, Alkoxyalkyl, -C(=S)R6, -C(=S)OR6, C(=S)NR6R7, -S(O)₂R6, -S(O)₂OR6, -P(O₂H)OR6, -CHR6OC(O)R7, -C₁₋₃-Alkyl-OC(O)R6, -C(OR6)R7R8, -CH(OR6)R7, -C(O)R6, -C(O)NR6R7 oder C(O)OR6 ist,
wobei R6, R7 und R8 jeweils unabhängig Wasserstoff, Alkyl, Cycloalkyl oder Aryl sind; und wobei die Alkylsubstituenten gegebenenfalls mit einem oder mehreren Halogenatomen substituiert sind;
R5 ist eine C₁₋₃-Alkylgruppe;
wobei die Verbindungen der Formel (I) als reine Enantiomere (R oder S) oder als Gemische hiervon, einschließlich Racematen, oder pharmazeutisch akzeptablen Salzen oder einem Metaboliten hiervon, zur Herstellung einer pharmazeutischen Zusammensetzung zur Prävention, Linderung oder Behandlung chronischer und/oder akuter Schmerzen vorliegen können,
wobei der Metabolit (S)-2-N-Propylamino-5-hydroxytetralin ist, und
wobei der Schmerz ausgewählt ist aus Schmerzen des Bewegungsapparates, einschließlich Rückenschmerzen, Fibromyalgie, myofasziale Schmerzen, Schmerzen während der Menstruation, Schmerzen bei Osteoarthritis, Schmerzen bei rheumatoider Arthritis, Schmerzen bei gastrointestinaler Entzündung, Schmerzen bei einer Entzündung des Herzmuskels, Schmerzen bei multipler Sklerose, Schmerzen bei Neuritis, Schmerzen bei Aids, Schmerzen während der Chemotherapie, Tumorschmerzen, Kopfschmerzen, CPS (chronisches Schmerzsyndrom), zentrale Schmerzen, neuropathische Schmerzen, wie zum Beispiel Trigeminusneuralgie, Herpes Zoster, Stumpfschmerzen, Phantomschmerzen, temporomandibuläre Gelenkerkrankung, Nervenschäden, post-herpetische Neuralgie oder neuropathische Schmerzen als Folge von Verletzungen, Amputationsinfektionen, metabolische Erkrankungen, neuropathische Schmerzen im Zusammenhang mit Diabetes, Pseudolisthesis, Hypothyreose, Urämie, Vitaminmangel oder Alkoholismus; sowie akute Schmerzen, wie Schmerzen nach Verletzungen, postoperative Schmerzen, Schmerzen bei akuter Gicht oder Schmerzen bei Operationen, wie zum Beispiel bei kieferchirurgischen Eingriffen.

2. Verwendung einer Verbindung nach Anspruch 1, wobei die pharmazeutische Zusammensetzung für nichtentzündliche Schmerzen des Bewegungsapparates vorgesehen ist.

3. Verwendung einer Verbindung nach Anspruch 2, wobei die pharmazeutische Zusammensetzung für Fibromyalgie, myofasziale Schmerzen oder Rückenschmerzen vorgesehen ist.

4. Verwendung einer Verbindung nach Anspruch 3, wobei die pharmazeutische Zusammensetzung für Fibromyalgie vorgesehen ist.

5. Verwendung einer Verbindung nach einem der vorhergehenden Ansprüche, wobei die pharmazeutische Zusammensetzung für die Reduzierung von muskulärer Hyperalgesie oder muskuläre Allodynie vorgesehen ist.

6. Verwendung nach einem der vorhergehenden Ansprüche, wobei A ein Wasserstoffatom oder eine Gruppe ist, ausgewählt aus 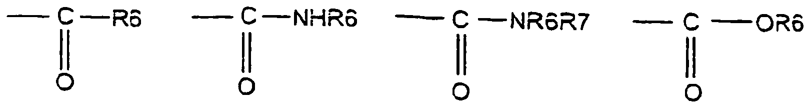 wobei R6 und R7 jeweils unabhängig C₁₋₁₂-Alkyl, Phenyl oder Methoxyphenyl sind.

7. Verwendung nach einem der vorhergehenden Ansprüche, wobei n 1, 2 oder 3 ist.

8. Verwendung nach einem der vorhergehenden Ansprüche, wobei R1 ausgewählt ist aus der Gruppe 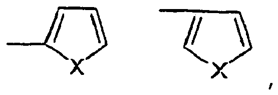 wobei X S, O oder NH ist.

9. Verwendung nach einem der vorhergehenden Ansprüche, wobei X ein Schwefelatom ist.

10. Verwendung nach einem der vorhergehenden Ansprüche, wobei R5 eine C₃-Alkylgruppe ist.

11. Verwendung nach einem der vorhergehenden Ansprüche, wobei R1 eine 2-Thienylgruppe ist, R3 und R4 jeweils Wasserstoff sind, R5 eine C₃-Alkylgruppe ist und n 2 ist.

12. Verwendung nach einem der vorhergehenden Ansprüche, wobei die Verbindung (+/-) 5,6,7,8-Tetrahydro-6-[propyl[2-(2-thienyl)ethyl]amino]-1-naphthol oder ein pharmazeutisch akzeptables Salz oder eine Metabolit hiervon ist, wobei der Metabolit (S)-2-N-Propylamino-5-hydroxytetralin ist.

13. Verwendung nach Anspruch 12, wobei die Verbindung das reine S-Enantiomer (Rotigotin) oder Rotigotinhydrochlorid ist.

14. Verwendung nach einem der vorhergehenden Ansprüche, wobei die pharmazeutische Zusammensetzung für die parenterale, transdermale oder mukosale Verabreichung vorgesehen ist.

15. Verwendung nach Anspruch 14, wobei die pharmazeutische Zusammensetzung für die transdermale Verabreichung vorgesehen ist.

16. Verwendung nach einem der vorhergehenden Ansprüche, wobei eine Verbindung der allgemeinen Formel (I) in einer Dosis von 0,05 bis 50 mg pro Tag verabreicht wird.

17. Verwendung nach einem der vorhergehenden Ansprüche, weiter umfassend die Verabreichung wenigstens eines weiteren Wirkstoffs.

18. Verwendung nach Anspruch 17, wobei der wenigstens eine weitere Wirkstoff ein Opioid, ein CGRP-Antagonist, ein NMDA-Rezeptorblocker, ein Cannabinoid, ein Bradykinin-Antagonist, Acetaminophen, ein NSAID, ein COX-2 selektiver Inhibitor, ein Sedativum, ein Antidepressivum, ein Beruhigungsmittel und/oder einen neuroprotektiven Wirkstoff umfasst.

19. Verwendung nach Anspruch 17, wobei der weitere Wirkstoff Dextromethorphan ist.

## Revendications

1. Utilisation d'un composé selon la formule (I) 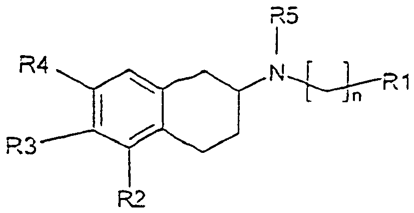 dans laquelle :
n est un nombre de 1 à 5 ;
R1 est choisi dans le groupe constitué de l'hydrogène, du 3-pyridyl, du 4-pyridyl, un phényle facultativement substitué, 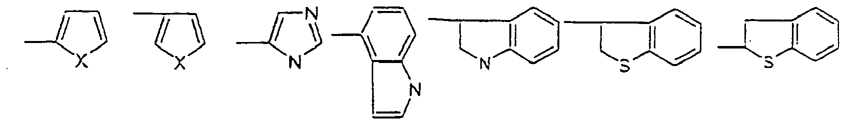
où X est S, O ou NH ;
R2 est un groupe -OA ; et R3 et R4 sont, chacun indépendamment, un atome d'hydrogène ou un groupe OA, dans lequel A est un atome d'hydrogène, un groupe alkyle, cycloalkyle, aryle, alkoxyalkyle, -C(=S)R6, -C(=S)OR6, C(=S)NR6R7, -S(O)₂R6, -S(O)₂OR6, -P(O₂H)R6, -P(O₂H)OR6, -CHR6OC(O)R7, -C₁₋₃ alkyle-OC(O)R6, -C(OR6)R7R8, -CH(OR6)R7, -C(O)R6, -C(O)NR6R7, ou C(O)OR6,
où R6, R7 et R8 sont, chacun indépendamment, un atome d'hydrogène ou un groupe alkyle, cycloalkyle ou aryle ; et où les substituants alkyle sont facultativement substitués avec un ou plusieurs atomes d'halogène ;
R5 est -C₁₋₃ alkyle ;
dans laquelle les composés de la Formule (I) peuvent être présents sous forme d'énantiomères purs (R ou S) ou tout mélange de ceux-ci, y compris les racémates, ou les sels pharmaceutiquement acceptables, ou un métabolite de ceux-ci, pour la préparation d'une composition pharmaceutique destinée à la prévention, à l'atténuation et/ou au traitement de la douleur chronique et/ou aiguë, dans laquelle le métabolite est la (S)-2-N-propylamino-5-hydroxytétraline, et dans laquelle la douleur est choisie parmi : douleur musculo-squelettique, y compris douleur du dos, fibromyalgie, douleur myofasciale, douleurs au cours de la menstruation, douleurs liées à l'arthrose, douleurs liées à la polyarthrite rhumatoïde, douleurs liées à une inflammation gastro-intestinale, douleurs liées à l'inflammation du muscle cardiaque, douleurs liées à la sclérose en plaques, douleurs liées à une névrite, douleurs liées au syndrome d'immuno-déficience acquise, douleurs liées à une chimiothérapie, douleurs liées à une tumeur, céphalées, syndrome douloureux chronique, douleur centrale, douleur neuropathique telle que la névralgie du trijumeau, zona, douleur du moignon, douleur du membre fantôme, pathologies de l'articulation temporo-mandibulaire, lésions des nerfs, névralgie post-herpétique ou douleur neuropathique rencontrée à la suite de blessures, d'infections après amputation, de problèmes métaboliques, douleurs neuropathiques associées au diabète, à la pseudesthésie, à l'hypothyroïdie, à l'urémie, à un déficit en vitamines ou à l'alcoolisme ; et douleur aiguë telles que douleurs après blessures, douleur post-opératoire, douleurs pendant une crise de goutte aiguë, ou douleurs au cours d'une opération, telle qu'une chirurgie de la mâchoire.

2. Utilisation d'un composé selon la revendication 1, dans laquelle la composition pharmaceutique est prévue pour une douleur musculo-squelettique non inflammatoire.

3. Utilisation d'un composé selon la revendication 2, dans laquelle la composition pharmaceutique est prévue pour une fibromyalgie, une douleur myofasciale ou une douleur du dos.

4. Utilisation d'un composé selon la revendication 3, dans laquelle la composition pharmaceutique est prévue pour une fibromyalgie.

5. Utilisation d'un composé selon l'une des revendications précédentes, dans laquelle la composition pharmaceutique est prévue pour réduire une hyperalgésie musculaire ou une allodynie musculaire.

6. Utilisation selon l'une quelconque des revendications précédentes, dans laquelle A est un atome d'hydrogène ou un groupe choisi parmi 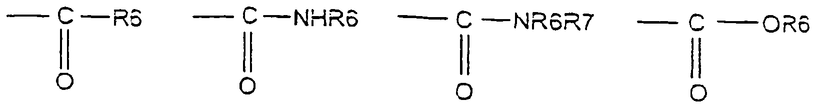 où R6 et R7 sont chacun indépendamment C₁₋₁₂ alkyle, phényle ou méthoxyphényle.

7. Utilisation selon l'une quelconque des revendications précédentes, où n est 1, 2 ou 3.

8. Utilisation selon l'une quelconque des revendications précédentes, où R1 est choisi parmi le groupe 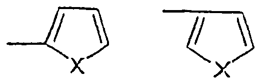 où X est S, O ou NH.

9. Utilisation selon l'une quelconque des revendications précédentes, où X est un atome de soufre.

10. Utilisation selon l'une quelconque des revendications précédentes, où R5 est un alkyle en C₃.

11. Utilisation selon l'une quelconque des revendications précédentes, où R1 est un 2-thiényle, R3 et R4 sont tous les deux un hydrogène, R5 est un alkyle en C₃ et n est 2.

12. Utilisation selon l'une quelconque des revendications précédentes, dans laquelle le composé est du (+/-)5,6,7,8-tétrahydro-6-[propyl[2-(2-thiényl)éthyl]amino]-1-naphtol ou un sel pharmaceutiquement acceptable ou un métabolite de celui-ci, où le métabolite est la (S)-2-N-propylamino-5-hydroxytétraline.

13. Utilisation selon la revendication 12, dans laquelle le composé est l'énantiomère S pur (rotigotine) ou le chlorhydrate de rotigotine.

14. Utilisation selon l'une quelconque des revendications précédentes, dans laquelle la composition pharmaceutique est destinée à une administration parentérale, transdermique ou par voie muqueuse.

15. Utilisation selon la revendication 14, dans laquelle la composition pharmaceutique est destinée à une administration transdermique.

16. Utilisation selon l'une quelconque des revendications précédentes, dans laquelle un composé de formule générale (I) est administré selon un dosage de 0,05 à 50 mg par jour.

17. Utilisation selon l'une quelconque des revendications précédentes, comprenant en outre l'administration d'au moins un autre agent actif.

18. Utilisation selon la revendication 17, dans laquelle ledit au moins un autre agent actif comprend un opioïde, un antagoniste du CGRP (peptide lié au gène de la calcitonine), un bloqueur du récepteur NMDA, un cannabinoïde, un antagoniste de la bradykinine, un acétaminophène, un AINS, un inhibiteur sélectif de la COX-2, un sédatif, un antidépresseur, un tranquillisant et/ou un agent neuroprotecteur.

19. Utilisation selon la revendication 17, dans laquelle ledit autre composé actif est le dextrométhorphane.
